# EUROPEAN PATENT APPLICATION

(11) **EP 4 624 516 A1**
(43) Date of publication of application: **01.10.2025**
(21) Application number: 24209346.6
(22) Date of filing: 29.10.2024
(51) Int. Cl.: C08G 69/10, C08G 69/40, C12N 15/88, A61K 9/51

(54) **NOVEL LIPIDS BASED ON OLIGO-Y-GLUTAMIC ACID DERIVATIVES AND LIPID NANOPARTICLES CONTAINING THE SAME, AND USES THEREOF**

(30) Priority: 27.03.2024 KR 20240041824
(71) Applicant: Korea Institute of Science and Technology, Seoul 02792 (KR)
(72) Inventor: Bang, Eun-Kyoung, 02792 Seoul (KR); KEUM, GYO CHANG, 02792 Seoul (KR); Yoo, Soyeon, 02792 Seoul (KR); Youn, Kounghwa, 02792 Seoul (KR); HWANG, IL KWON, 02792 Seoul (KR)
(74) Representative: advotec.

(57) **Abstract**

Provided are a novel lipid derivative compound including oligo-γ-glutamic acid, a lipid nanoparticle composition including the same, and the like. According to the present disclosure, the compound may form lipid nanoparticles by replacing PEGylated lipid, thereby preventing side effects such as anaphylaxis and exhibiting excellent in vivo stability.

## Description

### CROSS-REFERENCE TO RELATED APPLICATION

This application claims the benefit of Korean Patent Application No. 10-2024-0041824 filed on March 27, 2024, in the Korean Intellectual Property Office, the entire disclosure of which is incorporated herein by reference for all purposes.

### BACKGROUND

### 1. Field of the Invention

The present disclosure relates to novel lipids based on oligo-γ-glutamic acid, lipid nanoparticles containing the same, a vaccine composition further containing a therapeutic agent such as a nucleic acid in the lipid nanoparticles, and the like.

### 2. Description of the Related Art

Nucleic acid-based medicines, which began about 40 years ago by injecting plasmid DNA into the human body to help in producing deficient proteins, have then been reported to include various types, including antigene, decoy, antisense, siRNA, and miRNA, which inhibit the transcription and translation of genes. The nucleic acid-based medicines have attracted attention as personalized therapeutic agents by targeting DNA or RNA rather than proteins through complementary binding with a specific sequence of DNA or RNA. The nucleic acid-based medicines have been used not only as therapeutic agents, but also as preventive agents that defend against diseases by injecting genes capable of expressing antigens for specific diseases. Gene-based vaccines are divided into DNA vaccines, RNA vaccines, and viral vector vaccines. Among them, the RNA vaccines are types of expressing an antigen in the human body and inducing the formation of an antibody against the antigen by injecting messenger RNA (mRNA) encoding the antigen into the human body, and has advantages of being able to be developed quickly without concerns about infection of the viral vector-based vaccines or the potential risks of genetic mutations of the DNA vaccines, and thus have been in the spotlight as an effective response to COVID-19, which occurred in 2019.

Current lipid nanoparticles have been generally used in the form in which four components of ionizable lipid, phospholipid (helper lipid), cholesterol (structural lipid), and PEGylated lipid are mixed in a predetermined ratio. In particular, the PEGylated lipid has been widely used in pharmaceutical lipid nanoparticle (LNP) formulations of anticancer drugs such as doxorubicin, irinotecan, and cisplatin, as well as mRNA vaccines. However, hypersensitivity reactions, including anaphylaxis, and unexpected immune responses have been reported in relation to a plurality of PEG-containing formulations, and there is an urgent need to develop alternatives of PEGylated lipid that have fewer side effects and may maintain the stability in the body.

### SUMMARY

Embodiments provide a novel lipid derivative compound including oligo-γ-glutamic acid.

Embodiments also provide a lipid nanoparticle composition including the compound.

However, aspects of the present disclosure are not limited to the aforementioned aspects, and other aspects which are not mentioned may be clearly understood to those skilled in the art from the following description.

According to an aspect, there is provided a compound represented by Chemical Formula 1 below, a stereoisomer thereof, a racemate thereof, or a pharmaceutically acceptable salt thereof: 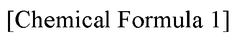 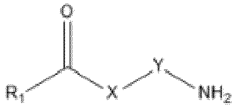
the X is absent or 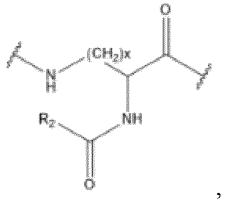
the x is an integer of 1 to 6,
the R₁ and R₂ are each independently saturated or unsaturated hydrocarbon having 6 to 22 carbon atoms,
the Y is a polymer represented by [AₐB_{b}C_{c}D_{d}EₑF_{f}]ₙ,
the A is 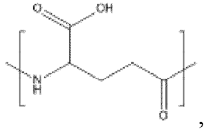
the B is 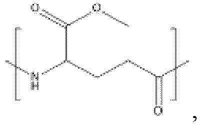
the C is 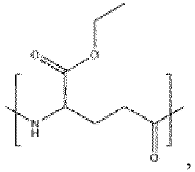
the D is 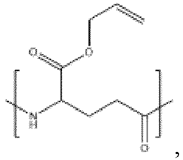
the E is 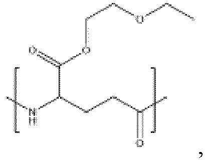
the F is 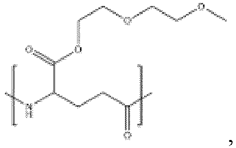 and
the a to f are integers of 0 to 24, the n is an integer of1 to 8, and the value of (a + b + c + d + e + f) * n is 1 to 24.

According to an embodiment, the R₁ and R₂ are each independently an alkyl group having 6 to 17 carbon atoms.

According to an embodiment, when the X is absent or 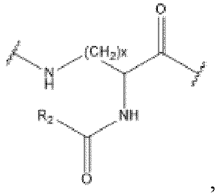 x may be 4, and the R₁ and R₂ may be straight-chain alkyl having 13 carbon atoms.

According to an embodiment, the n may be 5, the a and b may be each 1 or 2, and c to f may be 0.

According to an embodiment, the n may be 5, the a may be 2, c, e and f may be 0 or 1, at least one thereof may be 1, and b and d may be 0.

According to an embodiment, the compound may be at least one selected from the group consisting of Chemical Formula 1-1 to Chemical Formula 1-12 below:

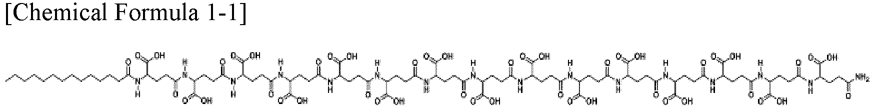

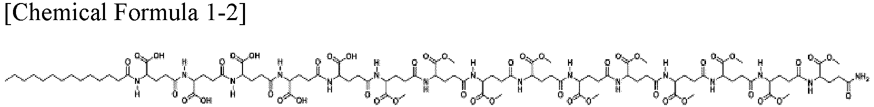

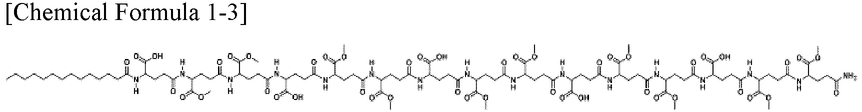

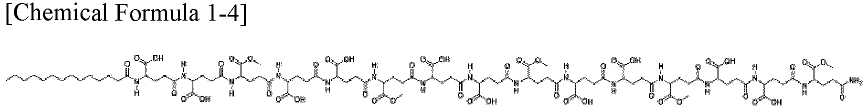

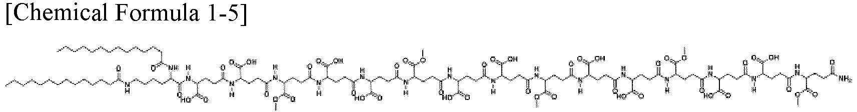

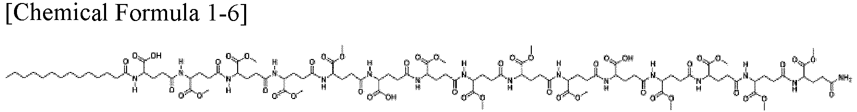

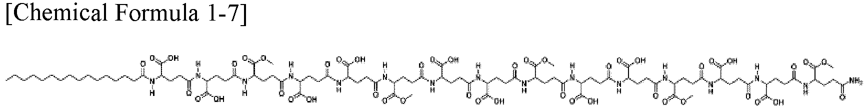

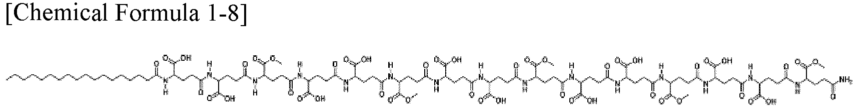

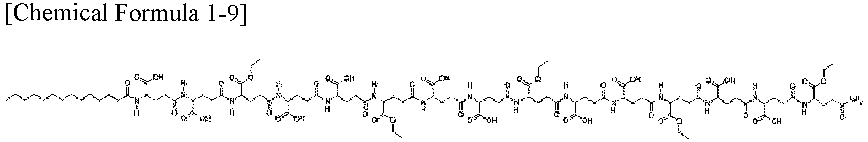

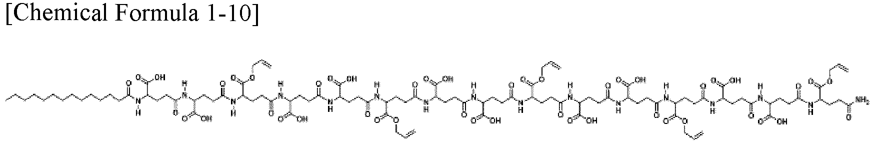

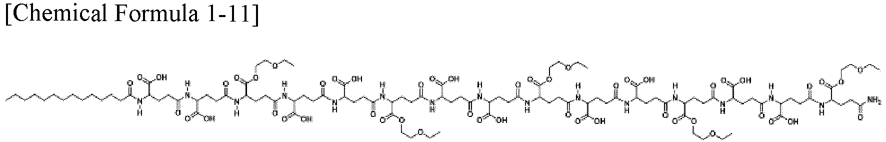

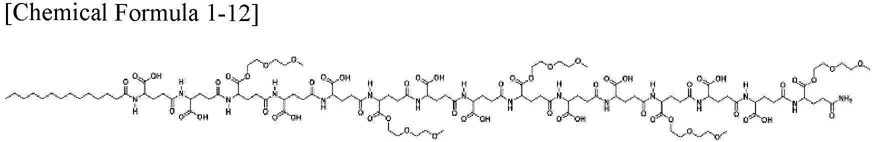

According to another example of the present disclosure, there is provided a lipid nanoparticle composition including the compound.

According to an embodiment, the compound may increase colloidal stability by replacing PEGylated lipid to prevent aggregation between particles.

According to an embodiment, the composition may further include at least one selected from the group consisting of ionizable lipid, helper lipid and structural lipid.

According to an embodiment, the helper lipid may be at least one selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18 : 0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanol amine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamin, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoyl phosphatidyl ethanolamine (DPPE), 1,2-dimyristoyl-sn-glycero-3-phosphoethanolamine (DMPE), 1-stearoyl-2-oleoyl-phosphatidyethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, and lysophosphatidylethanolamine (LPE).

According to an embodiment, the structural lipid may be at least one selected from the group consisting of cholesterol, bile acid derivatives including butyl lithocholate, cholanic acid derivatives, lithocholic acid derivatives, flavonoids, vitamin A and its derivatives, vitamin E, vitamin K, coenzyme Q10, and beta-carotene.

According to an embodiment, the composition may include all of ionizable lipid, helper lipid and structural lipid, and may include 1.5 to 15 mol% of the compound, 40 to 60 mol% of the ionizable lipid, 0 to 15 mol% of the helper lipid, and 25 to 40 mol% of the structural lipid.

According to an embodiment, the composition may include 10 mol% of the compound which is the compound represented by Chemical Formula 1-4, 45 to 50 mol% of the ionizable lipid, 5 to 7 mol% of the helper lipid, and 35 to 40 mol% of the structural lipid.

According to another aspect, the composition may include 1.5 mol% of the compound which is the compound represented by any one of Chemical Formula 1-9, Chemical Formula 1-11 or Chemical Formula 1-12, 50 mol% of the ionizable lipid, 10 mol% of the helper lipid, and 38.5 mol% of the structural lipid.

According to an embodiment, the compound may be any one selected from the group consisting of Chemical Formula 1-1 to Chemical Formula 1-12 below:

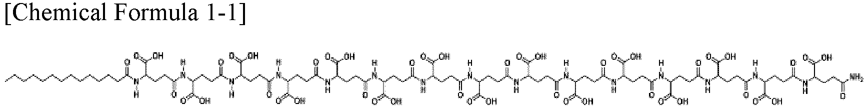

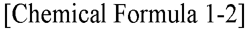

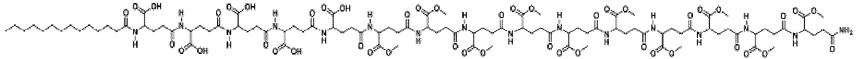

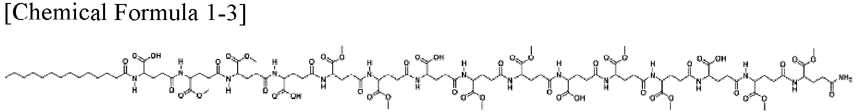

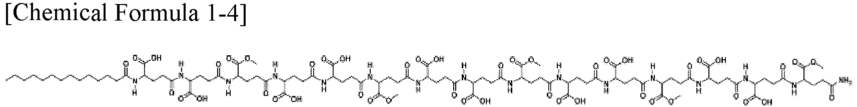

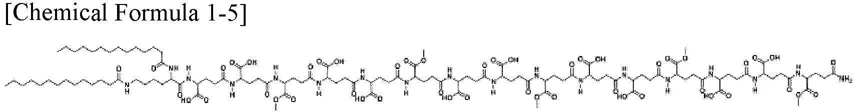

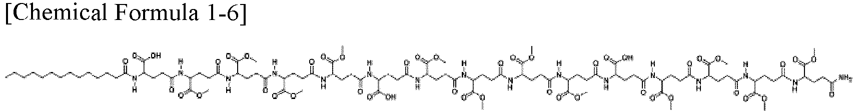

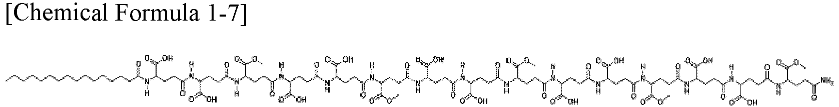

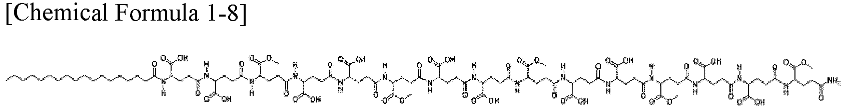

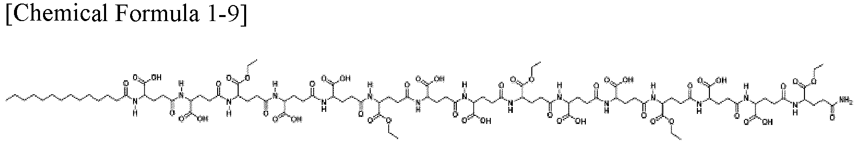

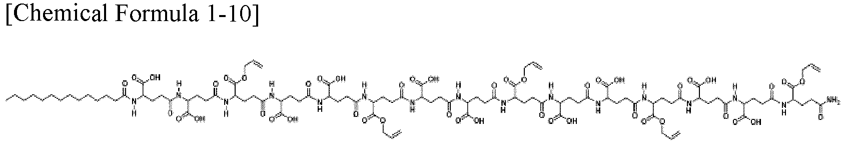

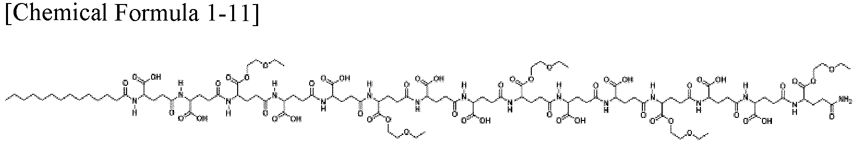

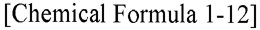

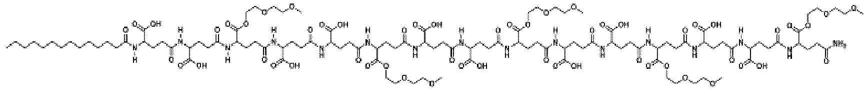

According to an embodiment, the lipid nanoparticle composition described above may include a therapeutic or preventive agent therein.

According to an embodiment, the therapeutic or preventive agent may be selected from the group consisting of interfering RNA (siRNA), asymmetric interfering RNA (aiRNA), microRNA (miRNA), dicer-substrate RNA (dsRNA), small hairpin RNA (shRNA), messenger RNA (mRNA) and mixtures thereof.

According to an embodiment, the therapeutic or preventive agent may be messenger RNA, and the lipid nanoparticles may desirably have a diameter of 70 to 200 nm and an internal zeta potential of -70 to -20 mV.

According to yet another example of the present disclosure, there is provided a vaccine composition including the lipid nanoparticle composition.

According to embodiments, it is possible to provide a novel lipid compound based on oligo-γ-glutamic acid, thereby forming lipid nanoparticles by replacing PEGylated lipid. The lipid nanoparticles including the compound of the present disclosure have fewer side effects such as allergic reactions and excellent in vivo stability compared to lipid nanoparticles including PEGylated lipid.

The effects of the present disclosure are not limited to the aforementioned effects, and other objects, which are not mentioned above, will be clearly appreciated by a person having ordinary skill in the art from the following description.

### BRIEF DESCRIPTION OF THE DRAWINGS

These and/or other aspects, features, and advantages of the invention will become apparent and more readily appreciated from the following description of embodiments, taken in conjunction with the accompanying drawings of which:
FIG. 1 shows results of performing gel retardation assay using lipid nanoparticles using derivatives of Chemical Formulas 1-1 to 1-5 to measure encapsulation efficiency of mRNA;
FIG. 2 shows results of performing gel retardation assay using lipid nanoparticles using a derivative of Chemical Formula 1-4 to measure encapsulation efficiency of mRNA;
FIG. 3 shows results of measuring intracellular mRNA delivery and translation efficiency of lipid nanoparticles using the derivative of Chemical Formula 1-4 using a flow cytometer;
FIG. 4 shows results of size analysis according to a refrigeration period of lipid nanoparticles using a derivative of Chemical Formula 1-4;
FIG. 5 shows results of gel retardation assay of lipid nanoparticles prepared with Oligo-γ-glutamic acid derivatives of Chemical Formulas 1-6 to 1-12 with a structure modified to optimize the structure of Chemical Formula 1-4;
FIG. 6 shows results of measuring intracellular mRNA delivery and translation efficiency of lipid nanoparticles OGA070, 073, and 076 using a flow cytometer; and
FIG. 7 shows results of performing gel retardation assay of lipid nanoparticles using derivatives of Chemical Formulas 1-3 and 1-4 to measure encapsulation efficiency of siRNA.

### DETAILED DESCRIPTION

The present inventors intended to invent and provide a novel lipid derivative compound based on glutamic acid to replace a PEGylated lipid, a lipid nanoparticle composition containing the same, a vaccine composition containing the same, and a method for preparing the same by conceiving that the PEGylated lipid, one of the components of lipid nanoparticles, increases colloidal stability to prevent aggregation between particles, thereby improving in vivo stability, but has an disadvantage of causing allergic reactions and anaphylaxis.

The present inventors provide a compound represented by Chemical Formula 1 below, a stereoisomer thereof, a racemate thereof, or a pharmaceutically acceptable salt thereof: 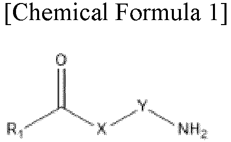
the X is absent or 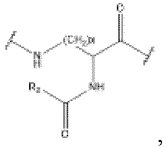
the x is an integer of 1 to 6,
the R₁ and R₂ are each independently saturated or unsaturated hydrocarbon having 6 to 22 carbon atoms,
the Y is a polymer represented by [AₐB_{b}C_{c}D_{d}EₑF_{f}]ₙ,
the A is 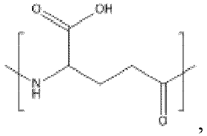
the B is 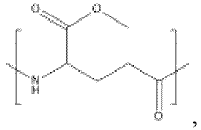
the C is 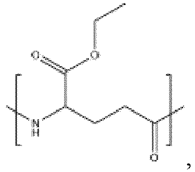
the D is 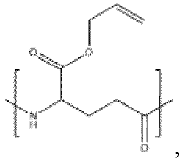
the E is 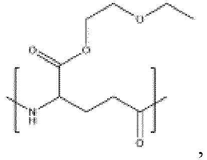
the F is 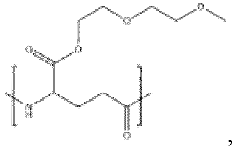 and
the a to f are integers of 0 to 24, the n is an integer of 1 to 8, and the value of (a + b + c + d + e + f) * n is 1 to 24.

As used in the present disclosure, the term `saturated hydrocarbon' means hydrocarbon composed of single bonds, and 'unsaturated hydrocarbon' means hydrocarbon having one or more double bonds or triple bonds.

As used in the present disclosure, the term 'polymer' may be a polymer formed by polymerization of single monomers or a polymer formed by polymerization of one or more different monomers as a copolymer. Desirably, the A to F may be formed by copolymerization, and since the value of (a + b + c + d + e + f) * n is 1 to 24, the compound of the present disclosure may include a total of 1 to 24 monomers, and most desirably, 15 monomers.

According to an embodiment, the R₁ and R₂ are each independently an alkyl group having 6 to 17 carbon atoms, and most desirably 13 to 15 carbon atoms.

As used in the present disclosure, the term "alkyl group" means that one hydrogen atom has been removed from an alkane, and may be desirably expressed as -(CH₂)ₐ-CH₃.

According to an embodiment, when the X is absent or 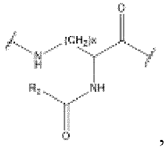 x may be 4, and the R₁ and R₂ may be straight-chain alkyl having 13 carbon atoms. The straight-chain alkyl refers to a structure in which several atoms are connected to each other in a long line a chemical structural formula.

According to an embodiment, the n may be 5, the a and b may be each 1 or 2, and c to f may be 0. Accordingly, the Y may be desirably (A₁B₂)₅ or (A₂B₁)₅, and most desirably (A₂B₁)₅. According to Example 3 or 4 of the present disclosure, when the copolymerization structure as described above was adopted, the most desirable properties for loading mRNA or siRNA were exhibited.

According to an embodiment, the n may be 5, the a may be 2, c, e and f may be 0 or 1, at least one thereof may be 1, and b and d may be 0. Therefore, the Y may be desirably (A₂C₁)₅, (A₂E₁)₅ or (A₂F₁)₅. According to Example 5 of the present disclosure, when the copolymerization structure as described above was adopted, desirable properties for loading mRNA were exhibited.

According to an embodiment, the compound may be at least one selected from the group consisting of Chemical Formulas 1-1 to 1-12 below:

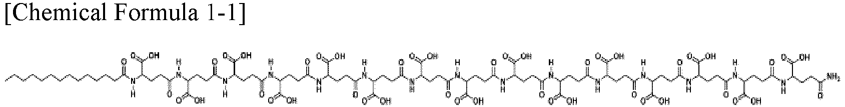

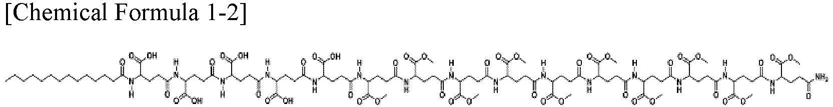

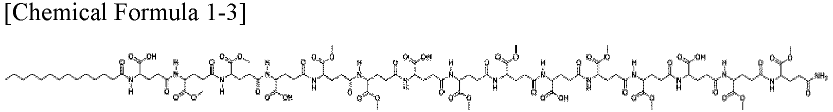

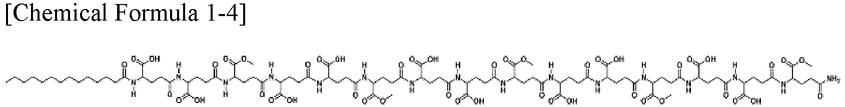

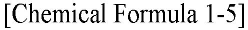

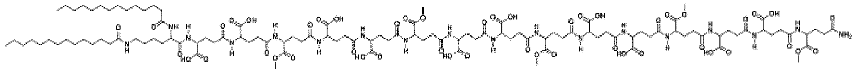

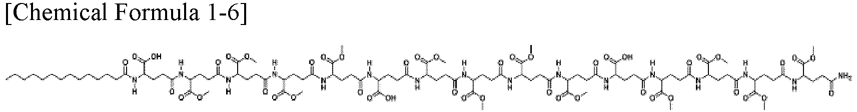

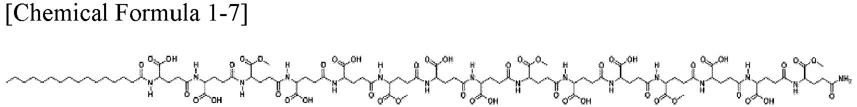

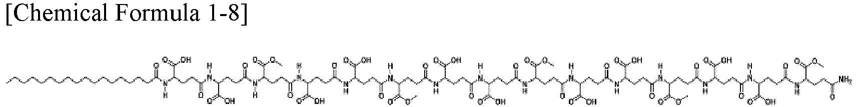

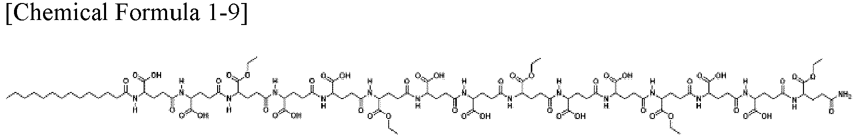

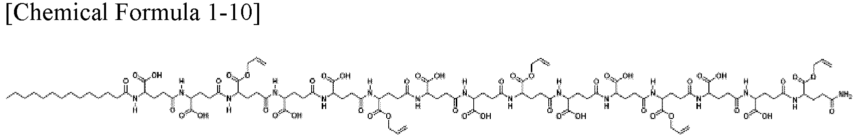

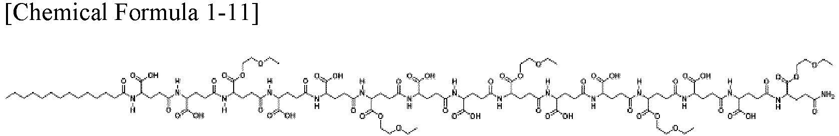

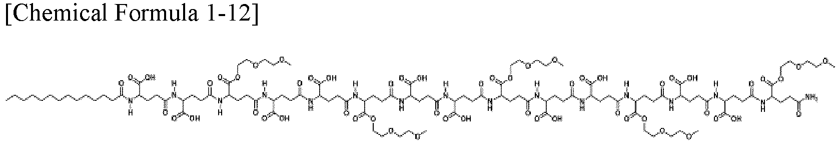

According to yet another example of the present disclosure, there is provided a lipid nanoparticle composition including the compound.

The lipid nanoparticles refer to nano-sized spherical particles composed of lipids.

According to an embodiment, the compound may increase colloidal stability by replacing PEGylated lipid to prevent aggregation between particles.

According to an embodiment, the composition may further include at least one selected from the group consisting of ionizable lipid, helper lipid and structural lipid, and desirably include all of ionizable lipid, helper lipid and structural lipid.

The ionizable lipid is an ionizable compound having properties similar to lipids and may play a role in efficiently encapsulating a drug (e.g., anionic drug and/or nucleic acid) into lipid nanoparticles through electrostatic interaction with the drug. The ionizable lipid may be protonated (positively charged) at a pH of less than pKa of the ionizable lipid, and may be substantially neutral at a pH of more than pKa. In an example, the lipid nanoparticles may include protonated ionizable lipid and/or neutral ionizable lipid. The ionizable lipid may be cationic lipid, and a non-limiting example thereof is SM-102.

The helper lipid may promote the fusion of lipid nanoparticles, and may be at least one selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18 : 0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanol amine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamin, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoyl phosphatidyl ethanolamine (DPPE), 1,2-dimyristoyl-sn-glycero-3-phosphoethanolamine (DMPE), 1-stearoyl-2-oleoyl-phosphatidyethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, and lysophosphatidylethanolamine (LPE), and most desirably DSPC.

The structural lipid refers to lipid that provides morphological rigidity to lipid charging within the lipid nanoparticles and is dispersed in the core and surface of the nanoparticles to enhance the stability of the nanoparticles. The structural lipid may be at least one selected from the group consisting of cholesterol, bile acid derivatives including butyl lithocholate, cholanic acid derivatives, lithocholic acid derivatives, flavonoids, vitamin A and its derivatives, vitamin E, vitamin K, coenzyme Q10 and beta-carotene, but most desirably cholesterol.

According to an embodiment, the composition may include all of ionizable lipid, helper lipid and structural lipid, and may include 1.5 to 15 mol% of the compound, 40 to 60 mol% of the ionizable lipid, 0 to 15 mol% of the helper lipid and 25 to 40 mol% of the structural lipid.

According to an embodiment, the composition may include 10 mol% of the compound which is the compound represented by Chemical Formula 1-4, 45 to 50 mol% of the ionizable lipid, 5 to 7 mol% of the helper lipid, and 35 to 40 mol% of the structural lipid.

According to another aspect, the composition may include 1.5 mol% of the compound which is the compound represented by any one of Chemical Formula 1-9, Chemical Formula 1-11 or Chemical Formula 1-12, 50 mol% of the ionizable lipid, 10 mol% of the helper lipid, and 38.5 mol% of the structural lipid.

As shown in Examples 4 and 7 below, optimal RNA loading efficiency and intracellular mRNA translation efficiency and stability were shown within the composition range described above.

According to an embodiment, the compound may be any one selected from the group consisting of Chemical Formulas 1-1 to 1-12 below:

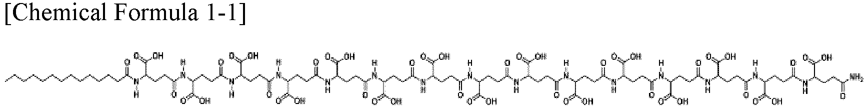

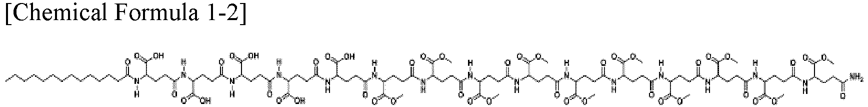

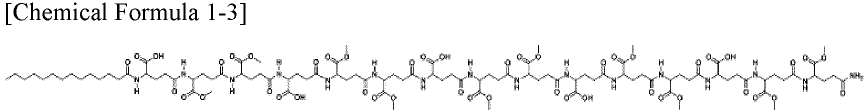

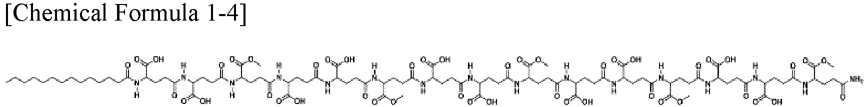

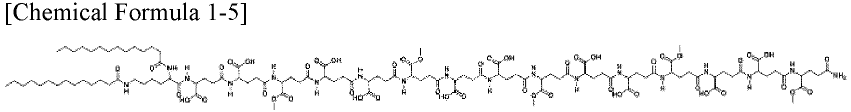

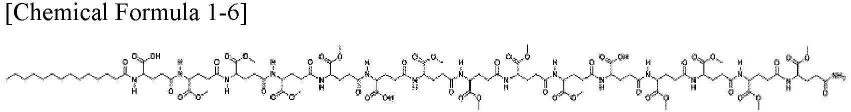

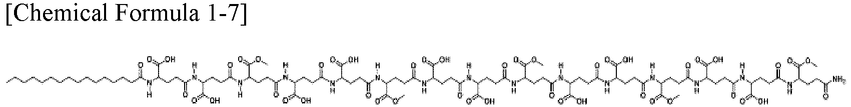

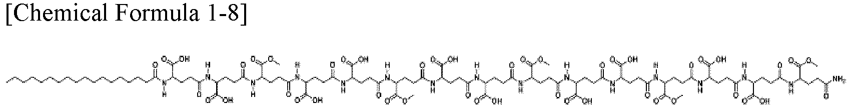

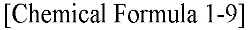

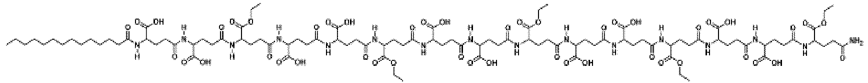

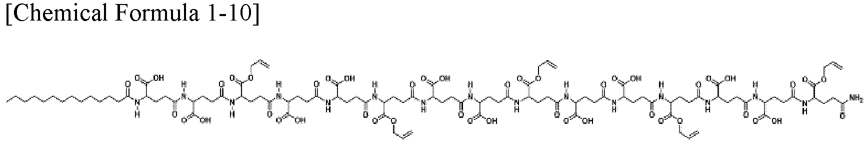

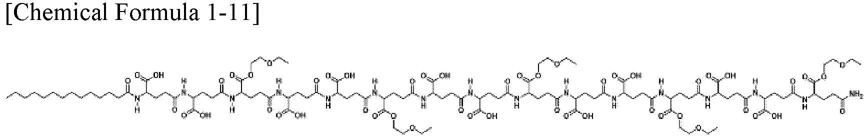

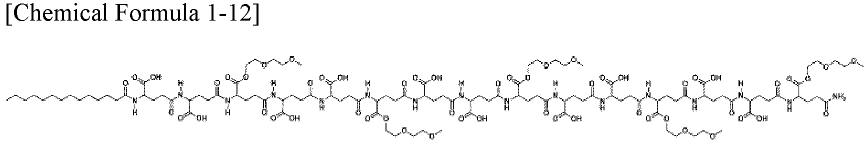

According to an embodiment, the lipid nanoparticle composition described above may include a therapeutic or preventive agent therein, and the therapeutic or preventive agent may desirably be selected from the group consisting of interfering RNA (siRNA), asymmetric interfering RNA (aiRNA), microRNA (miRNA), dicer-substrate RNA (dsRNA), small hairpin RNA (shRNA), messenger RNA (mRNA) and mixtures thereof, and most desirably messenger RNA. When the therapeutic or preventive agent is the messenger RNA, the lipid nanoparticles may desirably have a diameter of 70 to 200 nm and an internal zeta potential of -70 to -20 mV.

According to yet another example of the present disclosure, there is provided a vaccine composition including the lipid nanoparticle composition. The vaccine composition may desirably be an mRNA vaccine and may further include a pharmaceutically acceptable salt or an adjuvant.

The route of administration of the vaccine composition of the present disclosure may be, for example, an oral or parenteral route. Here, the parenteral route refers to a broad route of administration, and for example, includes intravenous, intraarterial, intraperitoneal, intramuscular, subcutaneous, intranasal, sublingual, intrathecal, inhalation, ocular, rectal, vaginal, ventricular administration, and the like.

In the case of formulating the composition, the formulation may be prepared by using a diluent or an excipient, such as a filler, an extender, a binder, a wetting agent, a disintegrating agent, and a surfactant, which are generally used.

Solid formulations for oral administration include tablets, pills, powders, granules, a capsule, troches, and the like, and the solid formulations may be prepared by mixing at least one excipient, for example, starch, calcium carbonate, sucrose, lactose, gelatin, or the like with at least one compound according to the present disclosure. Further, lubricants such as magnesium stearate talc may be used in addition to simple excipients. Liquid formulations for oral administration may correspond to suspensions, oral liquids, emulsions, syrups, and the like, and may include various excipients, for example, a wetting agent, a sweetener, an aromatic agent, a preserving agent, and the like, in addition to water and liquid paraffin which are commonly used simple diluents.

Formulations for parenteral administration include a sterile aqueous solution, a non-aqueous solution, a suspension, an emulsion, a lyophilizing agent, a suppository, etc.

As the non-aqueous solution and the suspension, propylene glycol, polyethylene glycol, vegetable oil such as olive oil, injectable ester such as ethyl oleate, and the like may be used. As a base of the suppository, witepsol, macrogol, Tween 61, cacao butter, laurinum, glycerol, gelatin, and the like may be used.

The composition according to the present disclosure is administered in a pharmaceutically effective amount. In the present disclosure, the 'pharmaceutically effective amount' refers to an amount enough to treat the disease at a reasonable benefit/risk ratio applicable to medical treatment. An effective amount level may be determined according to factors including the type and severity of a disease of a patient, the activity of a drug, the sensitivity to a drug, a time of administration, a route of administration, an emission rate, duration of treatment, and simultaneously used drugs, and other factors well-known in the medical field. The composition of the present disclosure may be administered as an individual therapeutic agent or in combination with other therapeutic agents, and may be administered sequentially or simultaneously with conventional therapeutic agents, and administered singly or multiply. It is important to administer an amount capable of obtaining a maximum effect with a minimal amount without side effects by considering all the factors, which may be easily determined by those skilled in the art.

The terms used in the examples are used for the purpose of description only, and should not be construed to be limited. The singular expression includes the plural expression unless the context clearly dictates otherwise. In the present disclosure, it should be understood that term "comprising" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which examples pertain. Terms which are defined in a generally used dictionary should be interpreted to have the same meaning as the meaning in the context of the related art, and are not interpreted as ideal or excessively formal meanings unless otherwise defined in the present disclosure.

The present disclosure may have various modifications and various Examples, and specific Examples will be hereinafter illustrated in the drawings and described in detail in the detailed description. However, the present disclosure is not limited to specific embodiment, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

### Example 1. Synthesis of Oligo-γ-glutamic acid derivatives

Oligo-γ-glutamic acid derivatives (12 types below) were designed as follows. After first designing a γ-glutamic acid monomer A and its derivative monomers B to F, C, E, and F, which were not commercially available, were synthesized as follows. The oligo-γ-glutamic acid derivatives basically consisted of 15 γ-glutamic acid monomers A to F having lipids composed of alkyl chains and repeating patterns. In the designed oligo-γ-glutamic acid derivatives, all monomers including synthesized monomers were provided to Dandicure Co., Ltd., and the synthesis was requested in compliance with a general solid-state peptide synthesis method.

### Example 1-1: Synthesis of Chemical Formula C

### Synthesis of 5-(tert-butyl) 1-ethyl (((9H-fluoren-9-yl)methoxy)carbonyl)-L-glutamate

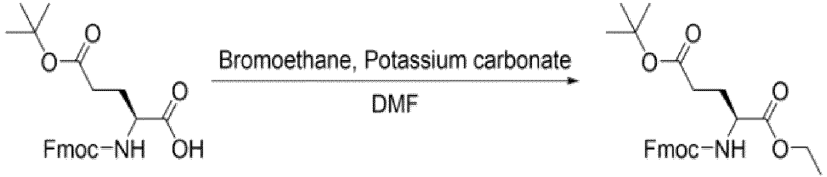

(S)-2-((((9H-fluoren-9-yl)methoxy)carbonyl)amino)-5-(tert-butoxy)-5-oxopentanoic acid (1.09 g) was dissolved in 20 mL of dimethylformaldehyde, and added with potassium carbonate (0.23 g) under strong stirring conditions. The reaction mixture was added with bromoethane (91 µl) and then stirred at room temperature for 8 hours. The reaction mixture was added with distilled water and a solvent was removed under a high vacuum. The reaction mixture was extracted into an organic layer by adding distilled water and ethyl acetate, and the organic layer was washed with a saturated sodium chloride aqueous solution. The organic layer was collected and the solvent was removed under a high vacuum. The reaction mixture was purified by column chromatography (SiO₂, dichloromethane → dichloromethane : methanol 9 : 1) to obtain a compound of 5-(tert-butyl) 1-ethyl (((9H-fluoren-9-yl)methoxy)carbonyl)-L-glutamate (1.06 g, 92%).

¹H NMR (400 MHz, Chloroform-*d*) δ 7.78 (dd, *J* = 7.6, 1.0 Hz, 2H), 7.61 (dd, *J* = 7.7, 7.6 Hz, 2H), 7.41 (dd, *J =* 7.5, 7.5 Hz, 2H), 7.33 (dd, *J =* 7.4, 7.4 Hz, 2H), 5.50 (d, *J =* 8.3 Hz, 1H), 4.59 - 4.31 (m, 2H+1H), 4.23 (q, *J* = 7.3 Hz, 2H+1H), 2.50 - 2.24 (m, 2H), 2.23 - 2.13 (m, 1H), 2.02 - 1.93 (m, 1H), 1.46 (s, 9H), 1.30 (t, *J* = 7.1 Hz, 6H).

¹³C NMR (101 MHz, Chloroform-*d*) δ 172.04, 155.95, 143.88, 143.69, 141.26, 127.67, 127.03, 125.09, 119.94, 80.80, 67.01, 61.60, 53.50, 47.11, 31.42, 28.03, 27.59, 14.13.

### Synthesis of (S)-4-((((9H-fluoren-9-yl)methoxy)carbonyl)amino)-5-ethoxy-5-oxopentanoic acid (C)

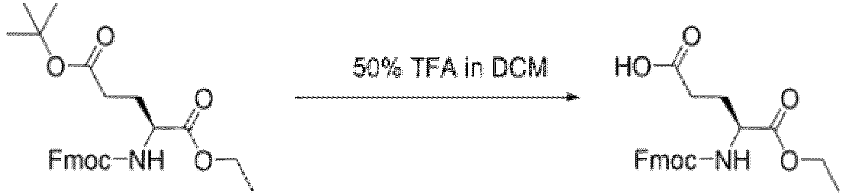

The 5-(tert-butyl) 1-ethyl (((9H-fluoren-9-yl)methoxy)carbonyl)-L-glutamate (807.7 mg) was dissolved in 9 mL of dichloromethane, and then added with trifluoroacetic acid (9 mL). The mixture was stirred in an ice bath for 3 hours, added with 20 mL of a saturated sodium bicarbonate aqueous solution and further stirred for 10 minutes. The organic layer was washed with distilled water and a saturated sodium chloride aqueous solution, and then the remaining water was removed with sodium sulfate. The reaction mixture was purified by column chromatography (SiO₂, dichloromethane → dichloromethane : methanol 94 : 6) to obtain a compound of Chemical Formula C (424 mg, 59.9%).

¹H NMR (400 MHz, DMSO-*d*₆) δ 7.89 (d, *J =* 7.5 Hz, 2H), 7.81 (d, *J =* 7.9 Hz, 1H), 7.72 (dd, *J* = 7.1, 2.2 Hz, 2H), 7.49 - 7.37 (m, 2H), 7.39 - 7.27 (m, 2H), 4.48 - 4.27 (m, 2H), 4.26 - 4.21 (m, 1H), 4.11 - 4.04 (m, 2H+1H), 2.46 - 2.25 (m, 2H), 2.07 - 1.91 (m, 1H), 1.88 - 1.70 (m, 1H), 1.17 (t, *J* = 7.1 Hz, 3H).

¹³C NMR (101 MHz, DMSO-*d*₆) δ 173.74, 172.15, 156.18, 143.85, 140.78, 127.71, 127.12, 125.26, 120.18, 65.71, 60.61, 53.17, 46.68, 30.00, 26.01, 14.09.

### Example 1-2: Synthesis of Chemical Formula E

### Synthesis of 5-(tert-butyl)1-(2-ethoxyethyl)(((9H-fluoren-9-yl)methoxy)carbonyl)-L-glutamate

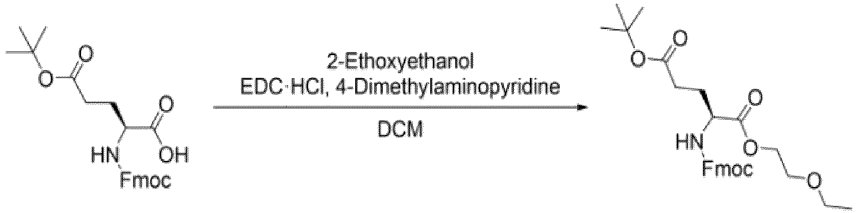

N-(3-dimethylaminopropyl)-N'-ethylcarbodiimide hydrochloride (451.9 mg) and 4-dimethylamino pyridine (29.1 mg) were dissolved in 85 mL of dichloromethane and then stirred in an ice bath under argon gas for 10 minutes. 2-Ethoxyethanol (230 µL) was mixed with 35 mL of dichloromethane, and slowly added dropwise to the reaction mixture in an ice bath under argon gas for 30 minutes. The reaction mixture was heated to room temperature and then stirred for 23.5 hours. The organic layer was washed with a saturated sodium bicarbonate aqueous solution and a saturated sodium chloride aqueous solution, and then the remaining water was removed with sodium sulfate. The reaction mixture was purified by column chromatography (SiO₂, dichloromethane → dichloromethane : methanol 93 : 7) to obtain a compound of 5-(tert-butyl) 1-(2-ethoxyethyl) (((9H-fluoren-9-yl)methoxy)carbonyl)-L-glutamate (510.9 mg, 87.0%).

¹H NMR (400 MHz, Chloroform-*d*) δ 7.77 (d, *J =* 7.5 Hz, 2H), 7.61 (dd, *J =* 7.7, 7.5 Hz, 2H), 7.41 (dd, *J =* 7.4, 7.4 Hz, 2H), 7.36 - 7.25 (m, 2H), 5.57 (d, *J =* 8.2 Hz, 1H), 4.47 - 4.34 (m, 2H+ 1H+ 1H), 4. 31 - 4.46 (m, 1H), 4.23 (t, J = 7.1 Hz, 1H), 3.64 (t, *J =* 4.8 Hz, 2H), 3.52 (q, *J* = 6.9 Hz, 2H), 2.42 - 2.26 (m, 2H), 2.26 - 2.12 (m, 1H), 2.08 - 1.94 (m, 1H), 1.46 (s, 9H), 1.21 (t, *J =* 7.0 Hz, 3H).

¹³C NMR (101 MHz, Chloroform-*d*) δ 172.02, 155.87, 143.85, 143.65, 141.21, 127.64, 127.01, 125.07, 119.91, 80.72, 67.98, 66.98, 66.55, 64.51, 53.47, 47.08, 31.33, 28.00, 27.54, 15.03.

### Synthesis of (S)-4-((((9H-fluoren-9-yl)methoxy)carbonyl)amino)-5-(2-ethoxyethoxy)-5-oxopentanoic acid (E)

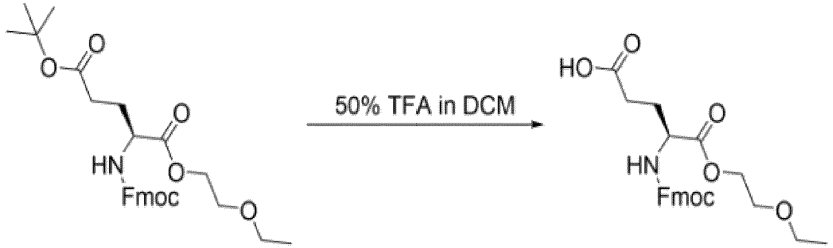

The 5-(tert-butyl) 1-(2-ethoxyethyl) (((9H-fluoren-9-yl)methoxy)carbonyl)-L-glutamate (749 mg) was dissolved in 7.5 mL of dichloromethane, and then added with trifluoroacetic acid (7.5 mL). The mixture was stirred in an ice bath for 3 hours, added with 15 mL of a saturated sodium bicarbonate aqueous solution and further stirred for 10 minutes. The organic layer was washed with distilled water and a saturated sodium chloride aqueous solution, and then the remaining water was removed with sodium sulfate. The reaction mixture was purified by column chromatography (SiO₂, dichloromethane → dichloromethane : methanol 94 : 6) to obtain a compound of Chemical Formula E (570 mg, 86.0%).

¹H NMR (400 MHz, Chloroform-*d*) δ 7.76 (d, *J =* 7.5 Hz, 2H), 7.60 (d, *J =* 7.5 Hz, 2H), 7.40 (dd, *J =* 7.5 Hz, 7.5 Hz, 2H), 7.32 (ddd, *J =* 7.5, 7.5 Hz, 1.4 Hz, 2H), 5.58 (d, *J =* 8.2 Hz, 1H), 4.52 - 4.38 (m, 2H+1H), 4.38 - 4.24 (m, 2H), 4.22 (t, *J =* 6.9 Hz, 1H), 3.64 (t, *J =* 4.8 Hz, 2H), 3.52 (q, *J =* 7.0 Hz, 2H), 2.54 - 2.39 (m, 2H), 2.28 - 2.20 (m, 1H), 2.06 - 1.97 (m, 1H), 1.20 (t, *J* = 7.0 Hz, 3H).

¹³C NMR (101 MHz, Chloroform-*d*) δ 177.72, 171.91, 156.04, 143.84, 141.32, 127.76, 127.12, 125.13, 120.03, 67.98, 67.12, 66.68, 64.68, 53.26, 47.15, 29.87, 27.54, 15.03.

### Example 1-3: Synthesis of Chemical Formula F

### Synthesis of 5-(tert-butyl) 1-(2-(2-methoxyethoxy)ethyl) (((9H-fluoren-9-yl)methoxy)carbonyl)-L-glutamate

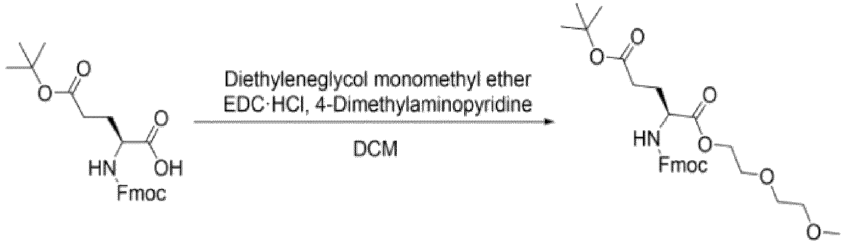

N-(3-dimethylaminopropyl)-N'-ethylcarbodiimide hydrochloride (465.0 mg) and 4-dimethylamino pyridine (31.5 mg) were dissolved in 85 mL of dichloromethane and then stirred in an ice bath under argon gas for 10 minutes. Diethyleneglycol monomethyl ether (280 µL) was mixed with 35 mL of dichloromethane, and then slowly added dropwise to the reaction mixture in an ice bath under argon gas for 30 minutes. The reaction mixture was heated to room temperature and then stirred for 17 hours. The organic layer was washed with a saturated sodium bicarbonate aqueous solution and a saturated sodium chloride aqueous solution, and then the remaining water was removed with sodium sulfate. The reaction mixture was purified by column chromatography (SiO₂, dichloromethane → dichloromethane : methanol 93 : 7) to obtain a compound of 5-(tert-butyl) 1-(2-(2-methoxyethoxy)ethyl) (((9H-fluoren-9-yl)methoxy)carbonyl)-L-glutamate (621 mg, quantitative yield).

¹H NMR (400 MHz, Chloroform-*d*) δ 7.77 (dd, *J =* 7.5, 1.1 Hz, 2H), 7.61 (dd, *J =* 7.7, 7.6 Hz, 2H7.47 - 7.37 (m, 2H), 7.32 (ddd, *J =* 7.4, 7.4, 0.9 Hz, 2H), 5.57 (d, *J =* 8.2 Hz, 1H), 4.52 - 4.36 (m, 2H+1H), 4.35 - 4.30 (m, 2H), 4.23 (t, *J =* 7.0 Hz, 1H), 3.72 (t, *J =* 4.8 Hz, 2H), 3.63 (dd, *J =* 5.7, 3.5 Hz, 2H), 3.53 (dd, *J =* 5.8, 3.4 Hz, 2H), 3.37 (s, 3H), 2.45 - 2.26 (m, 2H), 2.25 - 2.11 (m, 1H), 2.04 - 1.94 (m, 1H), 1.46 (s, 9H).

¹³C NMR (101 MHz, Chloroform-*d*) δ 172.04, 155.91, 143.87, 143.68, 141.25, 127.66, 127.03, 125.08, 119.93, 80.76, 71.82, 70.46, 68.82, 66.98, 64.43, 59.00, 53.49, 47.11, 31.38, 28.03, 27.53.

### Synthesis of (S)-4-((((9H-fluoren-9-yl)methoxy)carbonyl)amino)-5-(2-(2-methoxyethoxy)ethoxy)-5-oxopentanoic acid (F)

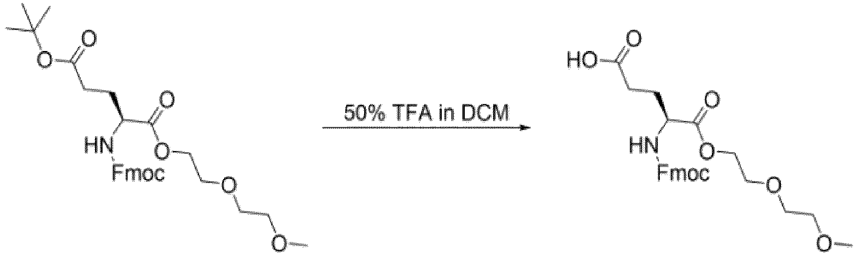

5-(tert-butyl) 1-(2-(2-methoxyethoxy)ethyl) (((9H-fluoren-9-yl)methoxy)carbonyl)-L-glutamate (824 mg) was dissolved in 7.5 mL of dichloromethane, and then added with trifluoroacetic acid (7.5 mL). The mixture was stirred in an ice bath for 2 hours, added with 15 mL of a saturated sodium bicarbonate aqueous solution and further stirred for 10 minutes. The organic layer was washed with distilled water and a saturated sodium chloride aqueous solution, and then the remaining water was removed with sodium sulfate. The reaction mixture was purified by column chromatography (SiO₂, dichloromethane → dichloromethane : methanol 94 : 6) to obtain a compound of Chemical Formula F (714 mg, 97.0%).

¹H NMR (400 MHz, Chloroform-*d*) δ 7.76 (d, *J =* 7.5 Hz, 2H), 7.60 (dd, *J =* 7.7, 7.6 Hz, 2H), 7.40 (ddd, *J =* 7.6, 7.6, 1.4 Hz, 2H), 7.32 (ddd, *J =* 7.4, 7.4, 1.4 Hz, 2H), 5.64 (d, *J =* 8.1 Hz, 1H), 4.56 - 4.33 (m, 2H+1H), 4.32 - 4.17 (m, 2H+1H), 3.70 (t, *J* = 4.7 Hz, 2H), 3.66 - 3.58 (m, 2H), 3.58 - 3.54 (m, 2H), 3.38 (s, 3H), 2.53 - 2.40 (m, 2H), 2.27 - 2.18 (m, 1H), 2.11 - 2.02 (m, 1H).

¹³C NMR (101 MHz,Chloroform-*d*) δ 176.56, 171.81, 155.90, 143.65, 141.24, 127.68, 127.04, 125.06, 119.94, 71.80, 70.13, 68.75, 67.00, 64.61, 58.84, 53.24, 47.08, 29.77, 27.34.

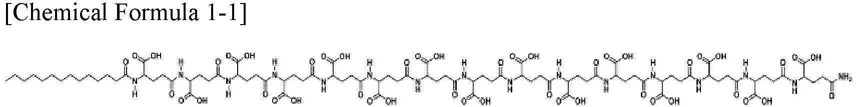

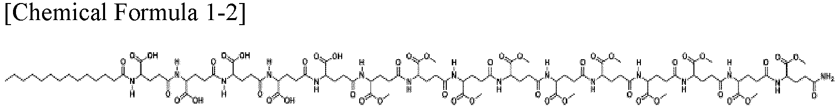

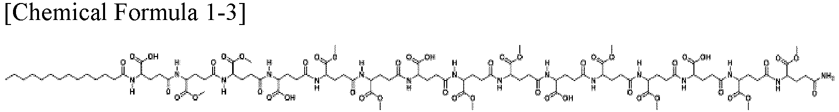

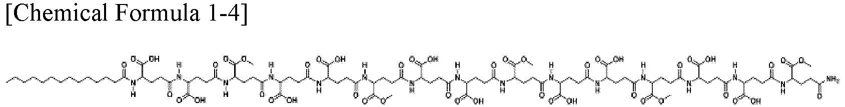

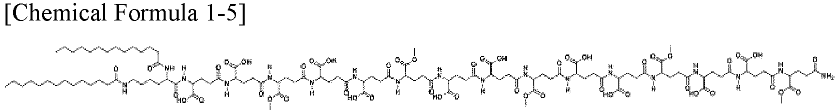

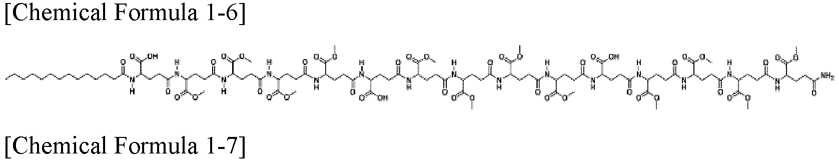

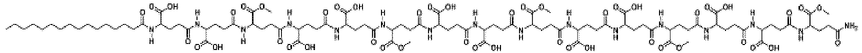

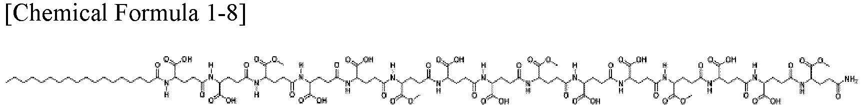

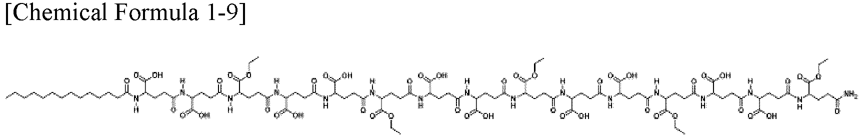

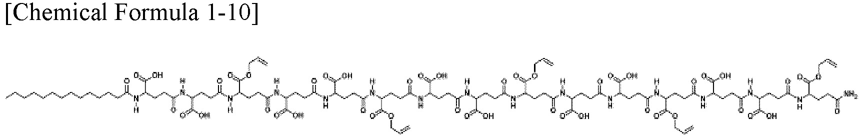

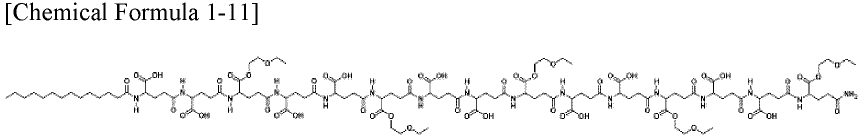

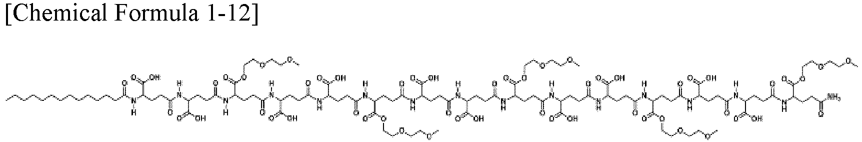

### Example 2. Preparation of lipid nanoparticles for mRNA delivery

Lipid nanoparticle carriers were prepared with compositions shown in Table 1 below by using the 12 types of oligo-γ-glutamic acid. The lipid nanoparticles may be prepared using a rotary hotplate stirrer (TS-100, Biosan), a low-volume laboratory mixer and emulsifier (NanoAssemblr Spark, Precision Nanosystems, Inc.), and a high-volume laboratory mixer and emulsifier (NanoAssemblr Ignite, Precision Nanosystems, Inc.), and in the lower order, the lipid nanoparticles are prepared in smaller and more uniform sizes. Accordingly, a first screening experiment was performed by manufacturing with the rotary hotplate stirrer, the experiments on intracellular mRNA delivery and translation efficiency were performed by manufacturing with the low-volume laboratory mixer and emulsifier, and a stability test of the final selected lipid nanoparticles was performed by manufacturing with the high-volume laboratory mixer and emulsifier.

The lipid nanoparticles were prepared by rapidly mixing an RNA solution (50 mM sodium citrate buffer, 110 mM sodium chloride, pH = 4.0) and a lipid mixture solution (ethanol, dimethyl sulfoxide) using a laboratory rotary hotplate stirrer (TS-100, Biosan), a low-volume laboratory mixer and emulsifier (NanoAssemblr Spark, Precision Nanosystems, Inc.) or a high-volume laboratory mixer and emulsifier (NanoAssemblr Ignite, Precision Nanosystems, Inc.), and then provided by changing the solvent to saline or phosphate buffered saline using a centrifugal filter tube (UFC5010, Amicon).

In the first lipid nanoparticle screening study, specifically, the lipid nanoparticles were prepared using a lipid mixture solution containing oligo-γ-glutamic acid derivatives Chemical Formulas 1-1 to 1-5 with the contents shown in Table 1 by a laboratory rotary hotplate stirrer (TS-100, Biosan). The sizes below represented diameters.

**[Table 1]**

| **LNP No.** | **Ionizabl e lipid (mol%)** | **Helper lipid (phospholipid) (mol%)** | **Cholesterol (mol%)** | **γ-OGA derivative lipid (mol%)** | **N/ P** | **size (nm)** | **PDI** | **Zeta potential (mV)** |
|---|---|---|---|---|---|---|---|---|
| **PEG-m** | SM-102 (50) | DSPC (10) | Cholesterol (38.5) | DMG-PEG2000 (1.5) | 6 | 158.3 ± 39.4 | 0.19 | 12.4 ± 0.5 |
| **OGA00 1** | SM-102 (50) | DSPC (10) | Cholesterol (38.5) | Chemical Formula 1-1 (1.5) | 6 | 1711 ± 315.5 | 0.93 | -10.0 ± 5.3 |
| **OGA00 2** | SM-102 (50) | DSPC (10) | Cholesterol (35) | Chemical Formula 1-1 (5) | 6 | 1410 ± 202.9 | 0.95 | -38.9 ± 1.9 |
| **OGA00 3** | SM-102 (50) | DSPC (10) | Cholesterol (30) | Chemical Formula 1-1 (10) | 6 | 129.3 ± 0.8 | 0.14 | -45.0 ± 1.0 |
| **OGA00 4** | SM-102 (50) | DSPC (10) | Cholesterol (38.5) | Chemical Formula 1-2 (1.5) | 6 | 3600 ± 306.9 | 1.00 | -6.4 ± 2.0 |
| **OGA00 5** | SM-102 (50) | DSPC (10) | Cholesterol (35) | Chemical Formula 1-2 (5) | 6 | 2750 ± 38.2 | 1.00 | -5.0 ± 4.6 |
| **OGA00 6** | SM-102 (50) | DSPC (10) | Cholesterol (30) | Chemical Formula 1-2 (10) | 6 | 1922 ± 570 | 0.93 | -12.2 ± 1.4 |
| **OGA00 7** | SM-102 (50) | DSPC (10) | Cholesterol (38.5) | Chemical Formula 1-3 (1.5) | 6 | 2961 ± 662.5 | 1.00 | -3.4 ± 1.0 |
| **OGA00 8** | SM-102 (50) | DSPC (10) | Cholesterol (35) | Chemical Formula 1-3 (5) | 6 | 2362 ± 553.7 | 1.00 | -11.1 ± 4.0 |
| **OGA00 9** | SM-102 (50) | DSPC (10) | Cholesterol (30) | Chemical Formula 1-3 (10) | 6 | 3356 ± 684.9 | 1.00 | -23.2 ± 2.5 |
| **OGA01 0** | SM-102 (50) | DSPC (10) | Cholesterol (38.5) | Chemical Formula 1-4 (1.5) | 6 | 2624 ± 263.2 | 1.00 | -11.0 ± 0.8 |
| **OGA01 1** | SM-102 (50) | DSPC (10) | Cholesterol (35) | Chemical Formula 1-4 (5) | 6 | 2801 ± 683.9 | 1.00 | -8.7 ± 1.9 |
| **OGA01 2** | SM-102 (50) | DSPC (10) | Cholesterol (30) | Chemical Formula 1-4 (10) | 6 | 207.2 ± 4.1 | 0.23 | -47.1 ± 0.3 |
| **OGA01 3** | SM-102 (50) | DSPC (10) | Cholesterol (38.5) | Chemical Formula 1-5 (1.5) | 6 | 2402 ± 164.0 | 1.00 | -17.2 ± 3.3 |
| **OGA01 4** | SM-102 (50) | DSPC (10) | Cholesterol (35) | Chemical Formula 1-5 (5) | 6 | 798.3 ± 96.7 | 0.70 | -45.0 ± 2.2 |
| **OGA01 5** | SM-102 (50) | DSPC (10) | Cholesterol (30) | Chemical Formula 1-5 (10) | 6 | 116.7 ± 2.3 | 0.28 | -54.5 ± 0.9 |
| **OGA01 6** | SM-102 (50) | DSPC (10) | Cholesterol (38.5) | Chemical Formula 1-6 (1.5) | 6 | 1179 ± 104.9 | 0.91 | -12.3 ± 3.9 |
| **OGA01 7** | SM-102 (50) | DSPC (10) | Cholesterol (35) | Chemical Formula 1-6 (5) | 6 | 2549 ± 166.6 | 1.00 | -6.2 ± 2.6 |
| **OGA01 8** | SM-102 (50) | DSPC (10) | Cholesterol (30) | Chemical Formula 1-6 (10) | 6 | 3150 ± 88.5 | 1.00 | -6.6 ± 4.3 |

### Example 3. Analysis of structural physicochemical properties of lipid nanoparticles and encapsulation efficiency of mRNA

The sizes, PDIs, and zeta potentials of 18 types of lipid nanoparticles, in which mol% of DMG-PEG2000 and cholesterol in a composition of lipid nanoparticles PEG-m containing DMG-PEG2000 were replaced with 1.5, 5, and 10 mol% of oligo-γ-glutamic acid derivatives Chemical Formulas 1-1 to 1-6, were measured using a dynamic light scattering particle size analyzer (Zetasizer, Malvern Panalytical Ltd.), which were shown in Table 1 above. Since the oligo-γ-glutamic acid derivatives were negatively charged at neutral pH, all 18 types of lipid nanoparticles had negative zeta potential values. Since the lipid nanoparticles were prepared using a rotary hotplate stirrer for rapid screening, lipid nanoparticles with a size of 300 nm or less and a PDI value of 0.3 or less were selected as suitable mRNA carriers, considering that the size and PDI value would decrease when prepared using a laboratory mixer and emulsifier. Lipid nanoparticles OGA003, 012, and 015 containing 10 mol% of Chemical Formulas 1-1, 1-4, and 1-5 exhibited suitable sizes and PDI values, respectively.

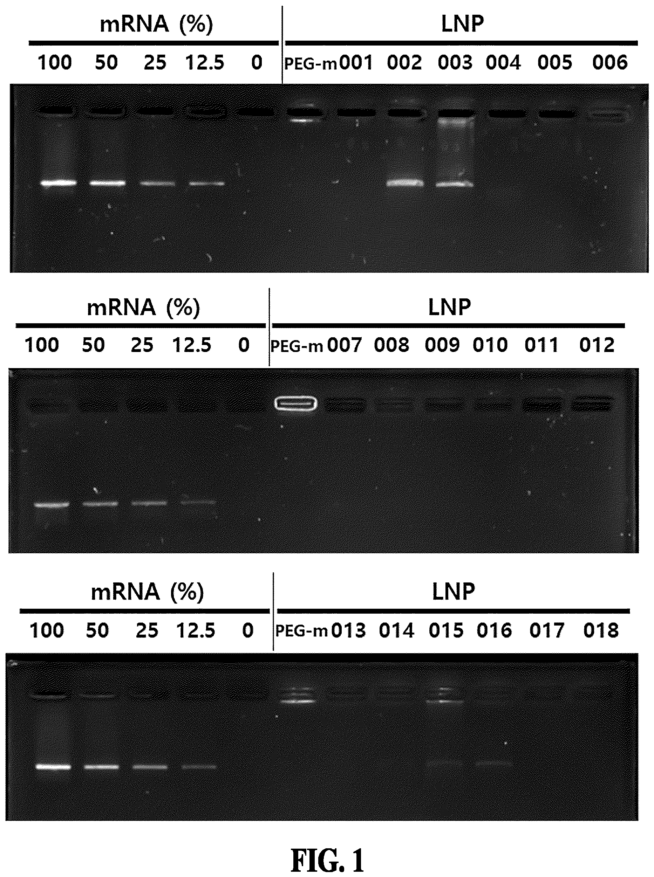

To measure the encapsulation efficiency of mRNA in mRNA-encapsulated lipid nanoparticles, gel retardation assay was performed, which was shown in FIG. 1. The gel retardation assay was performed by the following method. An mRNA standard solution and mRNA-encapsulated lipid nanoparticle samples were mixed at 5 : 1 with a loading buffer (36% glycerol) by concentration and then loaded into each well of a 1% agarose gel (containing Midori green dye). The agarose gel loaded with the samples was carefully placed into an electrophoresis device (mupid-2plus, ADVANCE) containing a 1X MOPS buffer solution so as to be fully immersed. After operating the electrophoresis device for about 5 minutes, the agarose gel was removed and fluorescence imaging was measured using a gel imaging system (Gel doc, Bio-Rad). Since fluorescence occurred when Midori Green dye bound to mRNA, it was meant that the higher the fluorescence intensity of bands that were shown on the same line as the mRNA standard solution, the lower the encapsulation efficiency of the lipid nanoparticles. When confirming three types of lipid nanoparticles with suitable sizes and PDIs for mRNA carriers, among lipid nanoparticles OGA003, 012, and 015 containing 10 mol% of Chemical Formulas 1-1, 1-4, and 1-5, OGA012 containing Chemical Formula 1-4 and OGA015 containing Chemical Formula 1-5 showed high mRNA encapsulation efficiency of 90% or higher, and particularly, the encapsulation efficiency of OGA012 was measured to be the highest at close to 100%. Lipid nanoparticles OGA003 containing Chemical Formula 1-1 showed mRNA encapsulation efficiency of about 50%.

As the results above, it was confirmed that the lipid nanoparticles containing 10 mol% of the oligo-γ-glutamic acid derivative represented by [Chemical Formula 1-4] in the present disclosure were the best by replacing PEG lipid, i.e. OGA012, which had appropriate size and PDI value and high mRNA encapsulation efficiency close to 100%. In Examples to be described below, the composition of lipid nanoparticles prepared using 10 mol% of the oligo-glutamic acid derivative of [Chemical Formula 1-4] was to be optimized.

### Example 4. Optimization of lipid nanoparticle composition for mRNA delivery

As described above, lipid nanoparticles were prepared using a laboratory low-volume mixer and emulsifier (NanoAssemblr Spark, Precision Nanosystems, Inc.) with various compositions so as to contain 10 mol% of the oligo-γ-glutamic acid derivative represented by [Chemical Formula 1-4], which was shown in Table 2 below.

**[Table 2]**

| **No.** | **Ionizable lipid (mol%)** | **Helper lipid (mol%)** | **Cholesterol (mol%)** | **γ-OGA derivative lipid (mol%)** | **N/P** | **Size (nm)** | **PDI** | **Zeta potential (mV)** |
|---|---|---|---|---|---|---|---|---|
| **PEG-m** | SM-102 (50) | DSPC (10) | Cholesterol (38.5) | DMG-PEG2000 (1.5) | 6 | 106.6 ± 1.4 | 0.06 | 3.9 ± 1.1 |
| **OGA012** | SM-102 (50) | DSPC (10) | Cholesterol (30) | Chemical Formula 1-4 (10) | 6 | 138.6 ± 3.3 | 0.10 | -36.0 ± 3.6 |
| **OGA046** | SM-102 (40) | DSPC (10) | Cholesterol (40) | Chemical Formula 1-4 (10) | 6 | 149.3 ± 1.2 | 0.11 | -31.7 ± 0.7 |
| **OGA047** | SM-102 (45) | DSPC (10) | Cholesterol (35) | Chemical Formula 1-4 (10) | 6 | 127.7 ± 1.8 | 0.10 | -36.4 ± 0.7 |
| **OGA048** | SM-102 (45) | DSPC (5) | Cholesterol (40) | Chemical Formula 1-4 (10) | 6 | 145.6 ± 0.2 | 0.16 | -38.6 ± 0.4 |
| **OGA049** | SM-102 (50) | DSPC (5) | Cholesterol (35) | Chemical Formula 1-4 (10) | 6 | 117.7 ± 0.5 | 0.07 | -36.9 ± 1.2 |
| **OGA050** | SM-102 (47) | DSPC (7) | Cholesterol (36) | Chemical Formula 1-4 (10) | 6 | 127.4 ± 0.8 | 0.09 | -32.1 ± 1.4 |
| **OGA054** | SM-102 (50) | DSPC (0) | Cholesterol (40) | Chemical Formula 1-4 (10) | 6 | 127.8 ± 1.6 | 0.08 | -45.4 ± 1.9 |

The sizes, PDIs, and zeta potentials of the prepared lipid nanoparticles were measured using a dynamic light scattering particle size analyzer (Zetasizer, Malvern Panalytical Ltd.), which were shown in Table 2. When the lipid nanoparticles were prepared using a low-volume laboratory mixer and emulsifier, the lipid nanoparticles were generally prepared at about 20 to 70 nm to be smaller and more uniform than those prepared using a rotary hotplate stirrer.

All of lipid nanoparticles PEG-m and OGA012, 046, 047, 048, 049, 050, and 054 with changed composition ratios of ionizable lipid, helper lipid, and cholesterol lipid, including a control PEG-m and excellent OGA012, exhibited sizes of 106.6 to 149.3 nm and PDIs of 0.06 to 0.16 suitable as mRNA carriers.

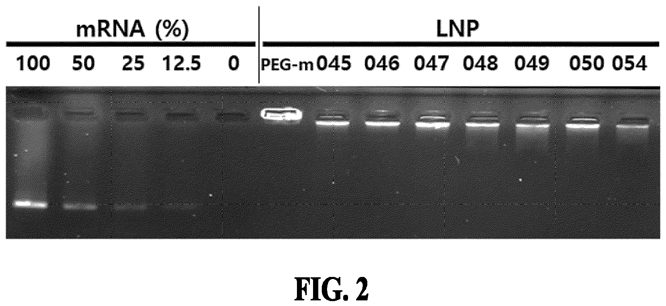

The results of performing gel retardation assay of the prepared LNPs were shown in FIG. 2. All of lipid nanoparticles 128-m and OGA012, 046, 047, 048, 049, 050, and 054 with changed composition ratios of ionizable lipid, helper lipid, and cholesterol lipid, including the control PEG-m and excellent OGA012, exhibited high mRNA encapsulation efficiency close to 100%.

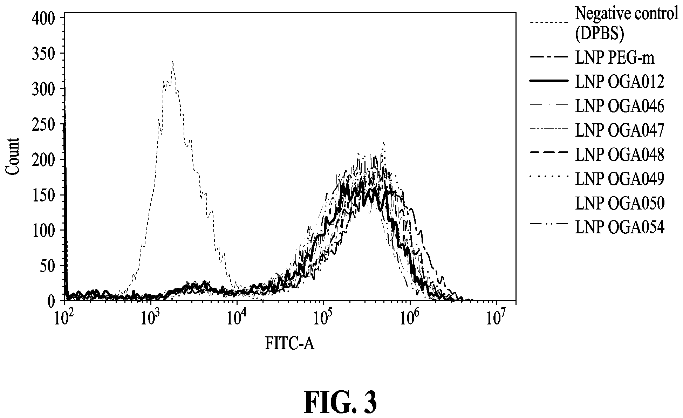

Similarly, the intracellular mRNA delivery and translation efficiency of the prepared LNP were measured using a flow cytometer (CytoFLEX, Beckman Coulter Life Sciences), and the results were shown in FIG. 3. Huh7 cells were cultured in a 24-well cell culture plate for one day, and then each well in which the cells were cultured was treated with lipid nanoparticles PEG-m and OGA012, 046, 047, 048, 049, 050, 054 encapsulated with GFP mRNA encoding green fluorescent protein (GFP) and DPBS as a negative control. After 24 hours of culture, the cells were detached from the cell culture plate and the cells expressing GFP were quantified using a flow cytometer. In FIG. 3, as the graph was biased to the right, there were more cells that expressed a high level of GFP. All of lipid nanoparticles OGA012, 046, 047, 048, 049, 050, and 054 containing 10 mol% of Chemical Formula 1-4 showed high intracellular delivery and translation efficiency of GFP mRNA similar to lipid nanoparticles PEG-m containing PEG. In particular, the lipid nanoparticles OGA048 to 050 prepared with 45 to 50 mol% of ionizable lipid, 5 to 7 mol% of helper lipid, 35 to 40% of cholesterol, and 10 mol% of Chemical Formula 1-4 exhibited the highest intracellular delivery and translation efficiency.

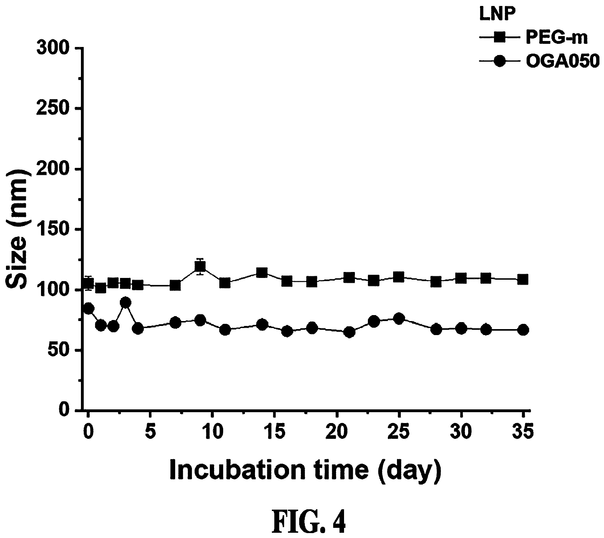

Finally, the stability according to long-term storage of the control group PEG-m (LNP prepared using PEG lipid) and lipid nanoparticles OGA050 was experimented as follows, and the results were shown in FIG. 4. The lipid nanoparticles were prepared using a high-volume laboratory mixer and emulsifier (NanoAssemblr Ignite, Precision Nanosystems, Inc.) and stored in a refrigerator at 4°C, and then the sizes of the lipid nanoparticles were measured at intervals of 2 to 3 days using a dynamic light scattering particle size analyzer (Zetasizer, Malvern Panalytical Ltd.). As a result, the lipid nanoparticles prepared using OGA instead of PEG of the present disclosure were maintained in sizes for 35 days or more to confirm excellent stability.

### Example 5. Study of optimizing structure of Chemical Formula 1-4

To optimize the structure of Chemical Formula 1-4, which was an oligo-γ-glutamic acid derivative selected as a substitute for PEG, oligo-γ-glutamic acid derivatives (7 types below) were designed by modifying the structure as follows. Since the oligo-γ-glutamic acid derivative of Chemical Formula 1-4 had a structure in which R₁ in Chemical Formula 1 was saturated hydrocarbon having 13 carbon atoms, oligo-γ-glutamic acid derivatives of Chemical Formulas 1-7 and 1-8 were designed by changing R₁ to saturated hydrocarbon structures of 15 and 17 carbon atoms, respectively. In addition, since the oligo-γ-glutamic acid derivative of Chemical Formula 1-4 had an oligomeric structure in which γ-glutamic acid (A) and a γ-glutamic acid derivative (B) were repeated five times in the form of AAB, oligo-γ-glutamic acid derivatives of Chemical Formulas 1-9 to 1-12 were designed by changing the γ-glutamic acid derivative (B) to γ-glutamic acid derivatives C to F, respectively. As described above, the lipid nanoparticles were prepared using a rotary hotplate stirrer (TS-100, Biosan) by containing the oligo-γ-glutamic acid derivatives represented by Chemical Formulas 1-7 to 1-12, which were shown in Table 3 below.

**[Table 3]**

| **No.** | **Ionizable lipid (mol%)** | **Helper lipid (mol%)** | **Choleste rol (mol%)** | **γ-OGA derivative lipid (mol%)** | **N/P** | **Size (nm)** | **PDI** | **Zeta potentia l (mV)** |
|---|---|---|---|---|---|---|---|---|
| **OGA064** | SM-102 (50) | DSPC (10) | Choleste rol (38.5) | Chemical Formula 1-7 (1.5) | 6 | 628.8 ± 341.4 | 0.62 | -30.7 ± 2.7 |
| **OGA065** | SM-102 (50) | DSPC (10) | Choleste rol (35) | Chemical Formula 1-7 (5) | 6 | 348.4 ± 9.2 | 0.39 | -53.4 ± 2.0 |
| **OGA066** | SM-102 (50) | DSPC (10) | Choleste rol (30) | Chemical Formula 1-7 (10) | 6 | 77.6 ± 2.0 | 0.29 | -58.4 ± 0.8 |
| **OGA067** | SM-102 (50) | DSPC (10) | Choleste rol (38.5) | Chemical Formula 1-8 (1.5) | 6 | 448.6 ± 194.5 | 0.51 | -17.1 ± 1.2 |
| **OGA068** | SM-102 (50) | DSPC (10) | Choleste rol (35) | Chemical Formula 1-8 (5) | 6 | 259.4 ± 7.4 | 0.21 | -51.7 ± 3.2 |
| **OGA069** | SM-102 (50) | DSPC (10) | Choleste rol (30) | Chemical Formula 1-8 (10) | 6 | 72.9 ± 1.0 | 0.16 | -48.9 ± 3.2 |
| **OGA070** | SM-102 (50) | DSPC (10) | Choleste rol (38.5) | Chemical Formula 1-9 (1.5) | 6 | 122.8 ± 0.5 | 0.10 | -39.8 ± 0.7 |
| **OGA071** | SM-102 (50) | DSPC (10) | Choleste rol (35) | Chemical Formula 1-9 (5) | 6 | 89.5 ± 3.1 | 0.21 | -39.2 ± 2.0 |
| **OGA072** | SM-102 (50) | DSPC (10) | Choleste rol (30 | Chemical Formula 1-9 (10) | 6 | 83.9 ± 1.3 | 0.22 | -40.4 ± 3.9 |
| **OGA079** | SM-102 (50) | DSPC (10) | Choleste rol (38.5) | Chemical Formula 1-10 (1.5) | 6 | 3361 ± 2480 | 0.94 | -45.4 ± 1.2 |
| **OGA080** | SM-102 (50) | DSPC (10) | Choleste rol (35) | Chemical Formula 1-10(5) | 6 | 397.4 ± 99.7 | 0.37 | -22.2 ± 3.9 |
| **OGA081** | SM-102 (50) | DSPC (10) | Choleste rol (30 | Chemical Formula 1-10 (10) | 6 | 172 ± 1.7 | 0.08 | -42.3 ± 2.1 |
| **OGA073** | SM-102 (50) | DSPC (10) | Choleste rol (38.5) | Chemical Formula 1-11 (1.5) | 6 | 169.6 ± 2.0 | 0.14 | -40.3 ± 1.2 |
| **OGA074** | SM-102 (50) | DSPC (10) | Choleste rol (35) | Chemical Formula 1-11 (5) | 6 | 104.2 ±1.5 | 0.24 | -40 ± 0.8 |
| **OGA075** | SM-102 (50) | DSPC (10) | Choleste rol (30 | Chemical Formula 1-11 (10) | 6 | 74.3 ± 1.4 | 0.17 | -38.3 ± 0.5 |
| **OGA076** | SM-102 (50) | DSPC (10) | Choleste rol (38.5) | Chemical Formula 1-12 (1.5) | 6 | 113.3 ± 0.7 | 0.08 | -38.1 ± 2.3 |
| **OGA077** | SM-102 (50) | DSPC (10) | Choleste rol (35) | Chemical Formula 1-12(5) | 6 | 117.9 ± 0.8 | 0.17 | -45.9 ± 2.5 |
| **OGA078** | SM-102 (50) | DSPC (10) | Choleste rol (30 | Chemical Formula 1-12 (10) | 6 | 95.3 ± 2.4 | 0.17 | -40.5 ± 1.5 |

The sizes, PDIs, and zeta potentials of the prepared lipid nanoparticles were measured using a dynamic light scattering particle size analyzer (Zetasizer, Malvern Panalytical Ltd.), which were shown in Table 3. It was confirmed that lipid nanoparticles OGA066, 068, 069, 070, 071, 072, 081, 073, 074, 075, 076, 077, and 078 were suitably prepared as mRNA carriers. In the case of Chemical Formulas 1-7 and 1-10, only the lipid nanoparticles OGA066, 081 containing 10 mol% showed suitable size and PDI values. In the case of Chemical Formula 1-8, only the lipid nanoparticles OGA068, 069 containing 5 and 10 mol% showed suitable size and PDI values. In the case of Chemical Formula 1-9, 1-11, and 1-12, all lipid nanoparticles OGA070, 071, 072, 071, 072, 073, 074, 075, and 076 containing 1.5, 5, and 10 mol% showed suitable sizes and PDI values.

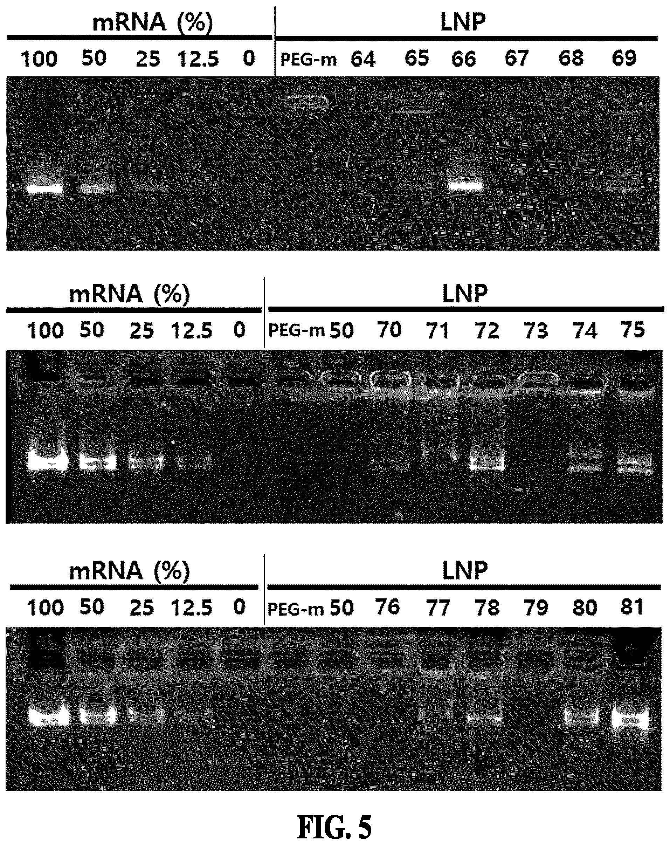

To measure the encapsulation efficiency of mRNA in mRNA-encapsulated lipid nanoparticles, gel retardation assay was performed, which was shown in FIG. 5. When confirming 13 types of lipid nanoparticles with suitable sizes and PDIs as mRNA carriers, lipid nanoparticles OGA068 containing 5 mol% of Chemical Formula 1-8 showed mRNA encapsulation efficiency of 90% or more, and lipid nanoparticles OGA069 containing 10 mol% of Chemical Formula 1-8 showed encapsulation efficiency of about 70%. The lipid nanoparticles OGA070, 071, 072, 073, 074, 075, 076, 077, and 078 containing 1.5, 5, and 10 mol% of Chemical Formulas 1-9, 1-11, and 1-12 showed lower mRNA encapsulation efficiency as the mol% increased, respectively. The lipid nanoparticles OGA070 containing 1.5 mol% of Chemical Formula 1-9, the lipid nanoparticles OGA073 containing 1.5 mol% of Chemical Formula 1-11, and the lipid nanoparticles OGA076 containing 1.5 mol% of Chemical Formula 1-12 showed high mRNA encapsulation efficiency of 90% or more.

As the results above, it was confirmed that the lipid nanoparticles containing 1.5 mol% of the oligo-γ-glutamic acid derivatives represented by [Chemical Formula 1-9], [Chemical Formula 1-11], and [Chemical Formula 1-12] in the present disclosure were the best by replacing PEG lipid, i.e. OGA070, OGA073, and OGA076, which had suitable size and PDI values and high mRNA encapsulation efficiency of 90% or more.

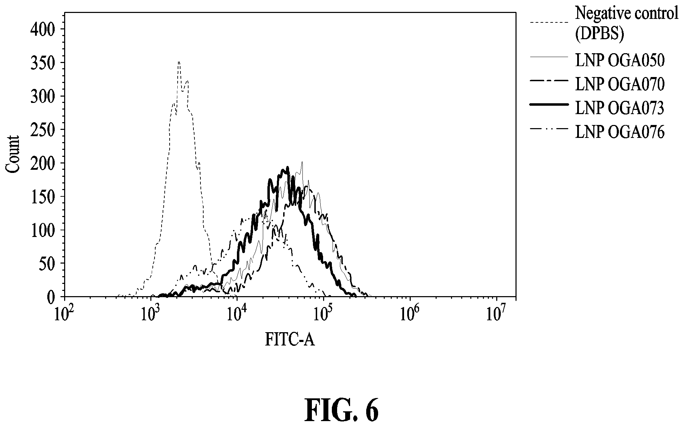

The intracellular mRNA delivery and translation efficiency of the lipid nanoparticles OGA070, 073, and 076 selected as excellent mRNA carriers were measured using a flow cytometer (CytoFLEX, Beckman Coulter Life Sciences), and the results were shown in FIG. 6. The lipid nanoparticles OGA070 containing 1.5 mol% of Chemical Formula 1-9 showed high intracellular mRNA delivery and translation efficiency similar to OGA050 containing 10 mol% of Chemical Formula 1-4. The lipid nanoparticles OGA 073 containing 1.5 mol% of Chemical Formula 1-11 showed slightly lower intracellular mRNA delivery and translation efficiency than OGA050 containing 10 mol% of Chemical Formula 1-4. The lipid nanoparticles OGA 076 containing 1.5 mol% of Chemical Formula 1-12 showed much lower intracellular mRNA delivery and translation efficiency than the lipid nanoparticles OGA 073 containing 1.5 mol% of Chemical Formula 1-11, but showed significantly higher intracellular mRNA delivery and translation efficiency than the negative control group.

In the case of the lipid nanoparticles containing the oligo-γ-glutamic acid derivatives of Chemical Formulas 1-9, 1-11, and 1-12, the lipid nanoparticles OGA070, 073, and 076 prepared with 50 mol% of ionizable lipid, 10 mol% of helper lipid, 38.5% of cholesterol, and 1.5 mol% of oligo-γ-glutamic acid derivatives exhibited suitable sizes and PDIs as mRNA carriers and high intracellular mRNA delivery and translation efficiency.

### Example 6. Preparation of lipid nanoparticles for siRNA delivery

As described in Example 2, the lipid nanoparticles were prepared using a laboratory rotary hotplate stirrer (TS-100, Biosan) by containing the oligo-γ-glutamic acid derivatives represented by Chemical Formulas 1-3 and 1-4, which were shown in Table 4 below.

**[Table 4]**

| **No.** | **Ionizable lipid (mol%)** | **Helper lipid (mol%)** | **Cholesterol (mol%)** | **γ-OGA derivative lipid (mol%)** | **N/P** | **Size (nm)** | **PDI** | **Zeta potential (mV)** |
|---|---|---|---|---|---|---|---|---|
| **PEG-si** | SM-102 (50) | DSPC (10) | Cholesterol (38.5) | DMG-PEG2000 (1.5) | 6 | 137.9 ± 1.3 | 0.21 | 14.6 ± 2.3 |
| **OGA027** | SM-102 (50) | DSPC (10) | Cholesterol (30) | Chemical Formula 1-3 (10) | 6 | 811.8 ± 162.5 | 0.63 | -28.1 ± 2.4 |
| **OGA030** | SM-102 (50) | DSPC (10) | Cholesterol (30) | Chemical Formula 1-4 (10) | 6 | 169.7 ± 1.7 | 0.06 | -46.8 ± 1.1 |
| **OGA037** | SM-102 (50) | DSPC (10) | Cholesterol (30) | Chemical Formula 1-3 / Chemical Formula 1-4 (9 / 1) | 6 | 604.5 ± 33.9 | 0.61 | -18.0 ± 2.5 |
| **OGA038** | SM-102 (50) | DSPC (10) | Cholesterol (30) | Chemical Formula 1-3 / Chemical Formula 1-4 (5/5) | 6 | 134.2 ± 1.6 | 0.09 | -38.6 ± 2.1 |
| **OGA039** | SM-102 (50) | DSPC (10) | Cholesterol (30) | Chemical Formula 1-3 / Chemical Formula 1-4 (1/9) | 6 | 125.6 ± 2.3 | 0.06 | -39.9 ± 1.3 |

### Example 7. Encapsulation of siRNA in lipid nanoparticles and characteristic analysis

The sizes, PDIs, and zeta potentials of the prepared lipid nanoparticles were measured using a dynamic light scattering particle size analyzer (Zetasizer, Malvern Panalytical Ltd.), which were shown in Table 4. Since the oligo-γ-glutamic acid derivatives were negatively charged at neutral pH, all 6 types of lipid nanoparticles had negative zeta potential values. Since the lipid nanoparticles were prepared using a rotary hotplate stirrer for rapid screening, lipid nanoparticles with a size of 300 nm or less and a PDI value of 0.3 or less were selected as suitable siRNA carriers, considering that the size and PDI value would decrease when prepared using a laboratory mixer and emulsifier. Lipid nanoparticles OGA027, 037, 038, 039, and 030 containing mol% ratios of Chemical Formulas 1-3 and 1-4 at 10 : 0, 9 : 1, 5 : 5, 1 : 9, and 0 : 10, respectively, showed that as the mol% of Chemical Formula 1-4 increased, the size and PDI values of the lipid nanoparticles decreased. The lipid nanoparticles OGA038, 039, and 030 containing 5 : 5, 1 : 9, and 0 : 10 mol% of Chemical Formulas 1-3 and 1-4 exhibited suitable sizes and PDI values.

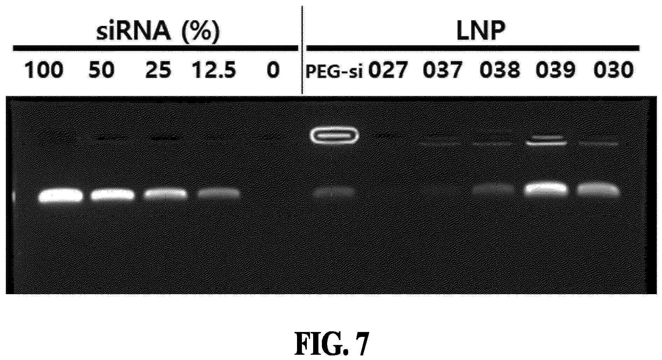

To measure the encapsulation efficiency of siRNA in FITC-siRNA-encapsulated lipid nanoparticles, gel retardation assay was performed, which was shown in FIG. 7. The gel retardation assay was performed by the following method. A FITC-siRNA standard solution and FITC-siRNA-encapsulated lipid nanoparticle samples were mixed at 5 : 1 with a loading buffer (36% glycerol) by concentration and then loaded into each well of a 1% agarose gel. The agarose gel loaded with the samples was carefully placed into an electrophoresis device (mupid-2plus, ADVANCE) containing a 1X MOPS buffer solution so as to be fully immersed. After operating the electrophoresis device for about 5 minutes, the agarose gel was removed and fluorescence imaging was measured using a gel imaging system (Gel doc, Bio-Rad). Since fluorescence occurred from a FITC fluorophore bound to siRNA, it was meant that the higher the fluorescence intensity of bands that were shown on the same line as the siRNA standard solution, the lower the encapsulation efficiency of the lipid nanoparticles. When confirming three types of lipid nanoparticles with suitable sizes and PDIs as siRNA carriers, among lipid nanoparticles OGA038, 039, and 030 containing 5 : 5, 1 : 9, and 0 : 10 mol% of Chemical Formulas 1-3 and 1-4, lipid nanoparticles OGA038 containing 5 mol% each of Chemical Formulas 1-3 and 1-4 showed high siRNA encapsulation efficiency of 90% or more. The lipid nanoparticles OGA039 containing 1 and 9 mol% of Chemical Formulas 1-3 and 1-4, respectively, were hardly encapsulated with siRNA, while the lipid nanoparticles OGA030 containing 10 mol% of Chemical Formula 1-4 showed encapsulation efficiency of 50% or more.

As the results above, it was confirmed that the lipid nanoparticles containing each 5 mol% of the oligo-γ-glutamic acid derivatives represented by [Chemical Formula 1-3] and [Chemical Formula 1-4] in the present disclosure were the best as siRNA carriers by replacing PEGlyated lipid, i.e. OGA038, which had appropriate size and PDI value and siRNA encapsulation efficiency of 90% or more.

As described above, although the examples have been described by the restricted drawings, various modifications and variations may be applied on the basis of the embodiments by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result may be achieved.

Therefore, other implementations, other embodiments, and equivalents to the appended claims fall within the scope of the claims to be described below.

## Claims

1. A compound represented by Chemical Formula I below, a stereoisomer thereof, a racemate thereof, or a pharmaceutically acceptable salt thereof: 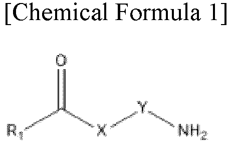
the X is absent or 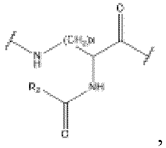
the x is an integer of 1 to 6,
the R₁ and R₂ are each independently saturated or unsaturated hydrocarbon having 6 to 22 carbon atoms,
the Y is a polymer represented by [AₐB_{b}C_{c}D_{d}EₑF_{f}]ₙ,
the A is 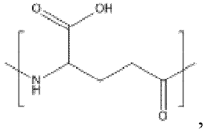 the B is 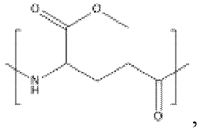
the C is 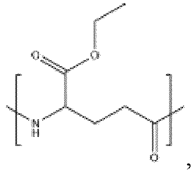
the D is 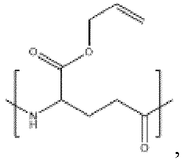
the E is 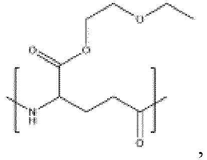
the F is 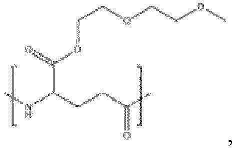
and
the a to f are integers of 0 to 24, the n is an integer of 1 to 8, and the value of (a + b + c + d + e + f) * n is 1 to 24.

2. The compound, the stereoisomer thereof, the racemate thereof, or the pharmaceutically acceptable salt thereof of claim 1, wherein the R₁ and R₂ are each independently an alkyl group having 6 to 17 carbon atoms.

3. The compound, the stereoisomer thereof, the racemate thereof, or the pharmaceutically acceptable salt thereof of claim 1 or 2, wherein the X is absent or 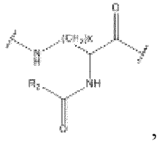 x is 4, and
the R₁ and R₂ are straight-chain alkyl having 13 carbon atoms.

4. The compound, the stereoisomer thereof, the racemate thereof, or the pharmaceutically acceptable salt thereof of one of claims 1 to 3, wherein the n is 5, the a and b are each 1 or 2, and c to f are 0.

5. The compound, the stereoisomer thereof, the racemate thereof, or the pharmaceutically acceptable salt thereof of one of claims 1 to 4, wherein the compound is at least one selected from the group consisting of Chemical Formula 1-1 to Chemical Formula 1-12 below:

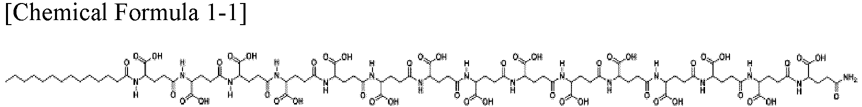

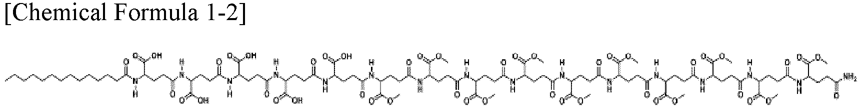

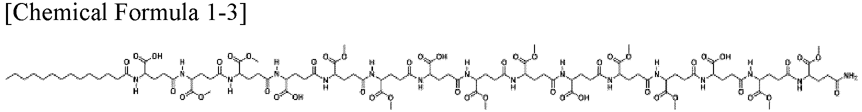

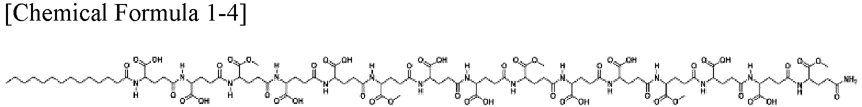

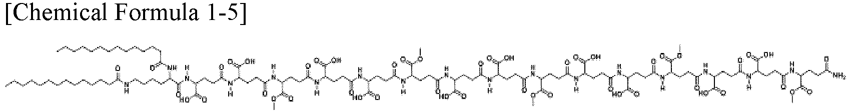

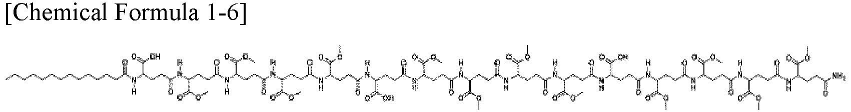

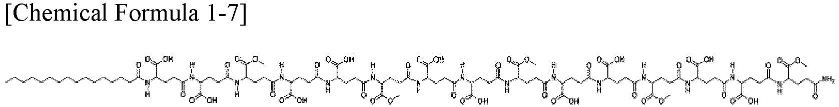

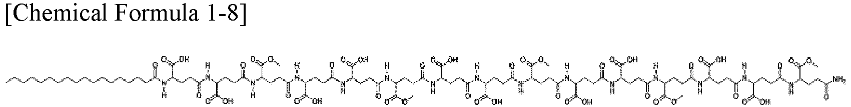

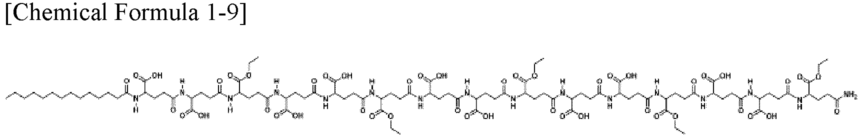

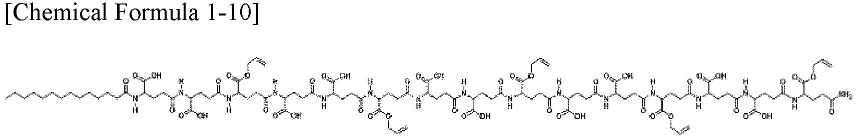

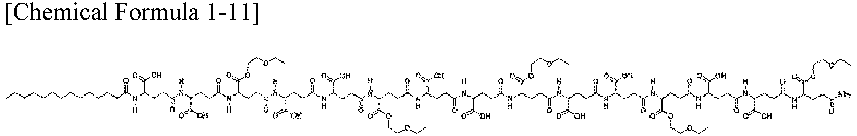

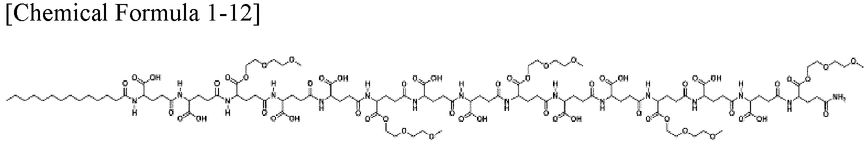

6. A lipid nanoparticle composition comprising the compound of one of claims 1 to 5.

7. The lipid nanoparticle composition of claim 6, wherein the compound increases colloidal stability by replacing PEGylated lipid to prevent aggregation between particles.

8. The lipid nanoparticle composition of claim 6 or 7, further comprising:
at least one selected from the group consisting of ionizable lipid, helper lipid and structural lipid.

9. The lipid nanoparticle composition of claim 8, wherein the helper lipid is at least one selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18 : 0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanol amine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamin, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoyl phosphatidyl ethanolamine (DPPE), 1,2-dimyristoyl-sn-glycero-3-phosphoethanolamine (DMPE), 1-stearoyl-2-oleoyl-phosphatidyethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoyl phosphatidylcholine, lysophosphatidylcholine, and lysophosphatidylethanolamine (LPE).

10. The lipid nanoparticle composition of claim 8 or 9, wherein the structural lipid is at least one selected from the group consisting of cholesterol, bile acid derivatives containing butyl lithocholate, cholanic acid derivatives, lithocholic acid derivatives, flavonoids, vitamin A and its derivatives, vitamin E, vitamin K, coenzyme Q10, and beta-carotene.

11. The lipid nanoparticle composition of one of claims 8 to 10, wherein the composition comprises all of ionizable lipid, helper lipid and structural lipid, and comprises 1.5 to 15 mol% of the compound, 40 to 60 mol% of the ionizable lipid, 0 to 15 mol% of the helper lipid and 25 to 40 mol% of the structural lipid.

12. The lipid nanoparticle composition of one of claims 8 to 11, wherein the composition comprises 10 mol% of the compound, 47 mol% of the ionizable lipid, 7 mol% of the helper lipid, and 36 mol% of the structural lipid, or comprises 1.5 mol% of the compound, 50 mol% of the ionizable lipid, 10 mol% of the helper lipid, and 38.5 mol% of the structural lipid.

13. The lipid nanoparticle composition of one of claims 6 to 12, wherein the compound is any one selected from the group consisting of Chemical Formula 1-1 to Chemical Formula 1-12 below:

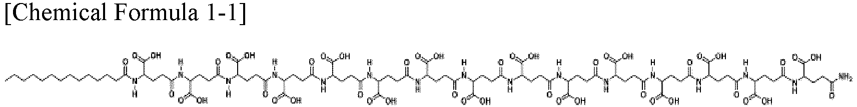

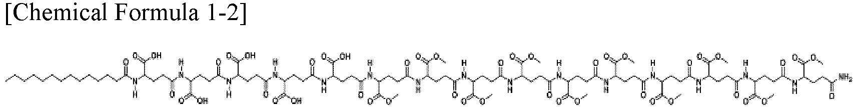

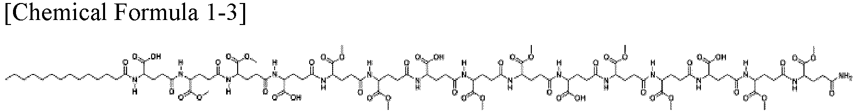

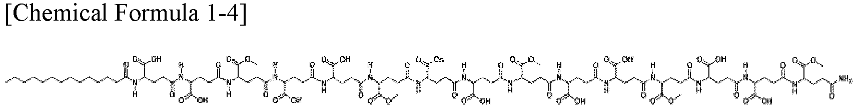

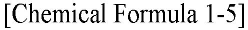

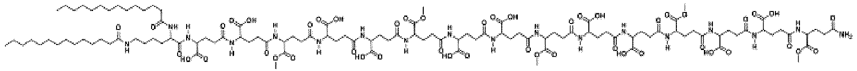

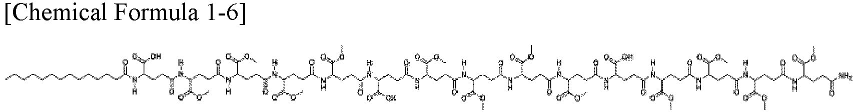

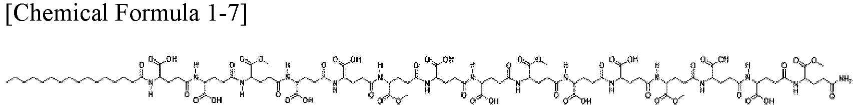

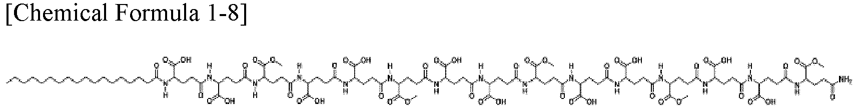

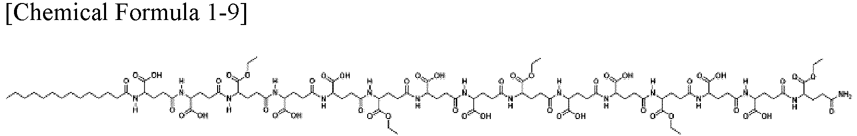

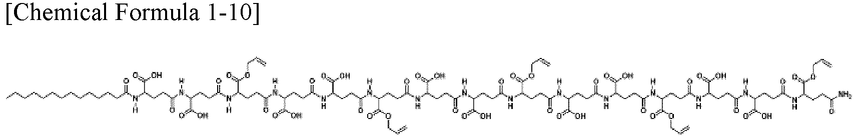

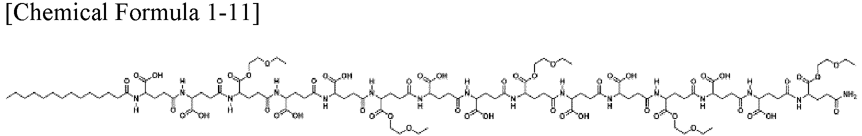

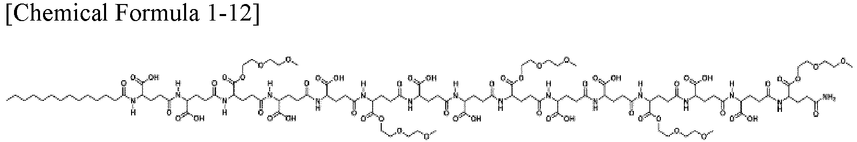

14. The lipid nanoparticle composition of any one of claims 8 to 13 wherein the lipid nanoparticle composition comprises a therapeutic or preventive agent therein.

15. The lipid nanoparticle composition of claim 14, wherein the therapeutic or preventive agent is selected from the group consisting of interfering RNA (siRNA), asymmetric interfering RNA (aiRNA), microRNA (miRNA), dicer-substrate RNA (dsRNA), small hairpin RNA (shRNA), messenger RNA (mRNA) and mixtures thereof.

16. The lipid nanoparticle composition of claim 15, wherein the therapeutic or preventive agent is messenger RNA, and
the lipid nanoparticles have a diameter of 70 to 200 nm and an internal zeta potential of -70 to -20 mV.

17. A vaccine composition comprising the lipid nanoparticle composition of one of claims 14 to 16.
